(19) Europäisches Patentamt
European Patent Office
Office européen des brevets

(11) Publication number: **0 480 041 A1**

# (12) EUROPEAN PATENT APPLICATION published in accordance with Art. 158(3) EPC

(21) Application number: **91905886.7**

(22) Date of filing: **22.03.91**

(86) International application number:
**PCT/JP91/00376**

(87) International publication number:
**WO 91/14705 (03.10.91 91/23)**

(51) Int. Cl.5: **C07K 15/12**, C12N 15/62, C12P 21/00, G01N 33/576, //(C12P21/00,C12R1:19)

(30) Priority: **28.03.90 JP 80520/90**

(43) Date of publication of application:
**15.04.92 Bulletin 92/16**

(84) Designated Contracting States:
**AT BE CH DE DK ES FR GB GR IT LI LU NL SE**

(71) Applicant: **KYOWA HAKKO KOGYO CO., LTD.**
**6-1, Ohtemachi 1-chome**
**Chiyoda-ku Tokyo 100(JP)**

(72) Inventor: **MORITA, Kazuki**
**1188, Shimotogari, Nagaizumi-cho**
**Sunto-gun, Shizuoka 411(JP)**
Inventor: **HASEGAWA, Mamoru**
**1-9-26, Katahira, Asao-ku**
**Kawasaki-shi, Kanagawa 229(JP)**
Inventor: **YOKOO, Yashiharu**
**3-4-17, Yokoyama**
**Sagamihara-shi, Kanagawa 229(JP)**
Inventor: **SATO, Moriyuki**
**2730-15, Naruse**
**Machida-shi, Tokyo 194(JP)**
Inventor: **SEKINE, Susumu**
**14, rue Plamet**
**F-75015 Paris(FR)**
Inventor: **SUGIMOTO, Seiji**
**3-9-10, Naka-machi**
**Machida-shi, Tokyo 194(JP)**
Inventor: **MORI, Hideharu**
**1188, Shimotogari, Nagaizumi-cho**
**Sunto-gun, Shizuoka 411(JP)**
Inventor: **ARIMA, Terukatsu**
**5-1, Harano-cho**
**Kagoshima-shi, Kagoshima 892(JP)**
Inventor: **YOSHIDA, Hajima**
**9-18, Isobe**
**Sagamihara-shi, Kanagawa 228(JP)**

(74) Representative: **Kinzebach, Werner, Dr.**
**Patentanwälte Reitstötter, Kinzebach und Partner Sternwartstrasse 4 Postfach 86 06 49**
**W-8000 München 86(DE)**

## (54) FUSED ANTIGEN POLYPEPTIDE.

(57) A fused antigen polypeptide comprising at least one hepatitis C related antigen polypeptide and a dog salmon growth hormone fused together via methionine, which is useful for detecting hepatitis C.

EP 0 480 041 A1

## FIELD OF THE INVENTION

The present invention relates to a fused antigen polypeptide in which an antigen polypeptide derived from a non-A, non-B hepatitis (type C hepatitis)-associated antigen is fused to a polypeptide heterologous to said antigen polypeptide, a recombinant plasmid containing, as an insert, a DNA coding for said fused antigen polypeptide, a method of producing fused antigen polypeptides which uses a microorganism containing any of said recombinant plasmids, and a method of detecting an anti-hepatitis C virus antibody which uses said fused antigen polypeptide. The antigen-antibody reaction is utilized in various medical and other scientific fields, and the fused antigen polypeptides of the invention are expected to be useful in a broad range of fields.

## BACKGROUND OF THE INVENTION

The virus called non-A, non-B hepatitis virus (NANBV) or hepatitis C virus (HCV) (hereinafter referred to as HCV) is thought to be a causative factor in type C hepatitis (hepatitis C) and serodiagnosis of HCV infection constitutes an important diagnostic technique for hepatitis C and for the prevention of HCV infection that may result from blood transfusion and so on. In said serodiagnosis, serum samples are tested for the presence or absence of an antibody to an HCV-associated antigen.

Choo et al. succeeded in cloning HCV from chimpanzee serum [Q.-L. Choo et al.: Science, 244, 359-362 (1989)]. Based on this success, a diagnostic reagent for hepatitis C has been developed [G. Kuo et al.: Science, 244, 362-364 (1989); European Patent Application No. 0318216].

HCV-associated genes have been cloned from sera of patients with hepatitis C [Arima et al.: Jikken Igaku (Experimental Medicine), Vol. 7, No. 2, 94-101 (1989); Arima et al.: Gastroenterologia Japonica, 24, 540-548 (1989)] and have been used in the diagnosis of hepatitis C. In the prior art mentioned above, fused antigen polypeptides each composed of a hepatitis C-associated antigen polypeptide and a heterologous protein are produced in microorganisms and used. Choo et al. used superoxide dismutase (hereinafter abbreviated as SOD) as the heterologous protein for fusion and Arima et al. used $\beta$-D-galactosidase (hereinafter abbreviated as $\beta$-gal) as such protein.

According to individuals, natural antibodies to these heterologous proteins may exist in their sera and these antibodies may react with the heterologous proteins. Therefore, high cut-off values should be employed for the diagnostic reagents. In addition, serum samples must be subjected to antibody absorption by adding relevant heterologous proteins prior to testing. This makes the procedure very complicated and may seriously affect the accuracy of diagnosis.

In such cases, a fused antigen polypeptide comprising an antigen polypeptide chemically bound to a carrier such as bovine serum albumin, a mixture of several antigen molecules, or a crude extract of pathogens is used. However, such procedures involve problems from the viewpoints of insuring constant availability and safety of fused antigen polypeptides. In an alternative method that has been proposed, direct expression of antigen polypeptides is sought in microorganisms. However, in many instances, such expression is difficult for reasons of the molecular weight, stability and toxicity of antigen polypeptides, hence it is impossible to supply antigen polypeptides constantly and at low cost.

The present inventors made investigations in an attempt to develop a method of producing antigen polypeptides useful for the detection of hepatitis C and, as a result, established a method of producing fused antigen polypeptides in which at least one hepatitis C-associated antigen polypeptide is fused to chum salmon growth hormone (hereinafter abbreviated as sGH) via methionine.

The present inventors further confirmed that when these fused antigen polypeptides are used for detecting anti-hepatitis C antibodies, said antibodies can be detected even in samples in which the detection methods so far reported fail to detect them and thus, the method of the present invention is superior to the prior art methods. The present invention has been completed based on the above findings.

## DESCRIPTION OF THE INVENTION

The present invention provides a fused antigen polypeptide resulting from the fusion of at least one hepatitis C-associated antigen-derived antigen polypeptide (hereinafter briefly referred to as hepatitis C-associated antigen polypeptide) to at least one polypeptide heterologous to said antigen polypeptide (hereinafter briefly referred to as heterologous polypeptide), a recombinant plasmid which contains, as an insert, a DNA coding for said fused antigen polypeptide, a method of producing a fused antigen polypeptide which comprises using a microorganism carrying said recombinant plasmid, and a method of detecting anti-hepatitis C virus antibodies (anti-HCV antibodies) using said fused antigen polypeptide.

BRIEF DESCRIPTION OF THE DRAWINGS

Fig. 1: Construction scheme for a plasmid named pSGHE1.
Fig. 2: Construction scheme for a plasmid named pSGHEC-2.
Fig. 3: Construction scheme for a plasmid named pSGHEC-14.
Fig. 4: Construction scheme for a plasmid named pSGHEC-18.
Fig. 5: Construction scheme for a plasmid named pSGHEC-18S.
Fig. 6: Construction scheme for a plasmid named pSGHEC-1414.
Fig. 7: Results of SDS-polyacrylamide gel electrophoresis of the fused antigen polypeptides produced by Escherichia coli transformants carrying the plasmids pSGHE1, pSGHEC-2, pSGHEC-14, pSGHEC-18S and pSGHEC-1414, respectively.
Fig. 8: Construction scheme for a plasmid named pSGHEC-14A.
Fig. 9: Construction scheme for a plasmid named pSGHEC-14B.
Fig. 10: Results of SDS-polyacrylamide gel electrophoresis of the fused antigen polypeptides produced by Escherichia coli transformants carrying the plasmids pSGHEC-14A and pSGHEC-14B, respectively.

In the practice of the invention, any HCV-associated antigen may be used as the hepatitis C-associated antigen polypeptide. Specifically, clone No. 2, clone No. 18 [Arima et al.: Gastroenterologia Japonica, 24, 540-548 (1989)] and clone No. 14, each cloned by Arima et al. as an HCV-associated antigen, may, inter alia, be employed.

The antigens that can be identified by these clone numbers are hereinafter referred to briefly as C-2, C-14 and C-18, respectively. The amino acid sequences of these antigens and the nucleotide sequences of DNAs coding for the antigens are shown under Sequence Identifier (SEQ ID) Nos. 1 to 3.

These three clones can be obtained as follows. In the first place, the blood plasma of a patient with hepatitis C is used as the starting material. RNA is extracted from this plasma and cDNA is prepared by the random primer method. A cDNA library is constructed from said cDNA using a cDNA cloning system λgt11 kit (Amersham). This library is transferred onto a nitrocellulose membrane and immunoscreening is performed using an acute hepatitis convalescent serum and the serum from a patient with chronic hepatitis each treated with Escherichia coli cell components for absorption.

The reactivity of these three clones for the patient serum is detected by the method described in Nippon Rinsho [Japanese Journal of Clinical Medicine, 48, 27-32 (1990)].

A sequence derived from the DNA base sequence for C-14 by partial substitution without causing any change in the amino acid sequence of C-14 is shown under SEQ ID NO: 41 and a sequence derived from the sequence No. 41 by partial deletion is shown under SEQ ID NO: 42. Thus, in the sequence No. 41, the 15th base A of the DNA base sequence for C-14 has been replaced by G, the 33rd base A by G, the 66th base C by T, the 69th base C by T, and the 99th base A by G. In the sequence No. 42, the 94th to 108th bases of the DNA base sequence of SEQ ID NO: 41 are missing.

The antigens described in Science, 244, 359-362 (1989), Science, 244, 362-364 (1989), and EP-A-0318216 may also be used as the HCV-associated antigen.

The heterologous polypeptide to be used in accordance with the invention may be any polypeptide provided that it does not specifically react with any of the human serum antibodies but can be expressed in a microorganism, in the form fused to a hepatitis C-associated antigen polypeptide. More specifically, the chum salmon growth hormone (sGH) polypeptide can preferably be used. The sequence of chum salmon growth hormone and the relevant genetic information and production technology, among others, are described in Proceedings of the National Academy of Sciences of the U.S.A., 82, 4306-4310 (1985) as well as in JP-A-61-15699, 61-93196, 61-93197 and 61-210100 (the term "JP-A" used herein means an unexamined published Japanese patent application).

The fused antigen polypeptide can be provided by the steps of inserting a DNA coding for a fused antigen polypeptide consisting of an HCV-associated antigen and a heterologous polypeptide having a molecular weight of not less than 2,000, joined together preferably via methionine, into a vector DNA by the recombinant DNA technology without involving reading frame shifting, using the thus-constructed recombinant plasmid to transform a microorganism, cultivating the transformant microorganism to cause formation and accumulation of said fused antigen polypeptide in the culture, and harvesting said fused antigen polypeptide from said culture.

The vector DNA may be of any kind only if it allows expression of the insert DNA in a microorganism. Thus, plasmids which have an appropriate promoter, for example the tryptophan (trp) or lactose (lac) promoter, and allow insertion of the desired DNA downstream from said promoter and in which the distance between the Shine-Dalgarno sequence and the translation initiation codon has been adjusted to an appropriate length, for example 6 to 18 bases, can preferably be used.

As specific examples of the vector DNA which are preferred, there may be mentioned pGEL1 (FERM BP-629; JP-A-61-93197), pKYP10 (JP-A-58-110600) and pGHA2 (FERM BP-400; JP-A-60-221091), among others.

As for the microorganism, any microorganism may be employed provided that the DNA coding for the fused antigen polypeptide of this invention can be expressed and that said polypeptide can be formed and accumulated in the culture. Microorganisms belonging to the species Escherichia coli are preferred.

The procedure for production of certain fused antigen polypeptides from chum salmon growth hormone and an HCV-associated antigen by joining them together is described below in further detail.

The fused antigen polypeptides in which chum salmon growth hormone is bound to an HCV-associated antigen via a methionine residue are produced by taking the following steps.

Steps 1 to 6 are involved in the production of the fused antigen polypeptides sGHE1, sGHEC-2, sGHEC-14, sGHEC-18S and sGHEC-1414, while steps 7 to 9 are involved in the production of the fused antigen polypeptides sGHEC-14A and sGHEC-14B.

[Step 1] Synthesis of HCV-associated antigen and linker genes

First, DNAs represented by SEQ ID NO: 4, 5, 6, 7, 8, 9, 10, 11, 12, 13, 14, 15, 16, 17, 18 and 19 are chemically synthesized.

The synthesis of these DNAs is performed by the solid-phase phosphoamidite method using an automatic DNA synthesizer.

A plasmid containing a double-stranded DNA fragment identifiable by SEQ ID NO: 20 (hereinafter referred to as gene-20) is constructed using a double-stranded DNA formed from the DNA identifiable by SEQ ID NO: 4 (hereinafter referred to as DNA-4) and the DNA identifiable by SEQ ID NO: 5 (hereinafter referred to as DNA-5).

(In SEQ ID NO: 20, the lines directed to "EcoRI" each indicates a site of cleavage by the restriction enzyme EcoRI. Hereinafter the same shall apply.)

Similarly, a plasmid containing a double-stranded DNA fragment identifiable by SEQ ID NO: 21 (hereinafter referred to as gene-21) is constructed by joining a double-stranded DNA formed from a DNA having SEQ ID NO: 6 (hereinafter referred to as DNA-6) and a DNA having SEQ ID NO: 7 (hereinafter referred to as DNA-7) to a double-stranded DNA formed from a DNA having SEQ ID NO: 8 (hereinafter referred to as DNA-8) and a DNA having SEQ ID NO: 9 (hereinafter referred to as DNA-9), using ligase.

Similarly, a plasmid containing a double-stranded DNA fragment represented by SEQ ID NO: 22 (hereinafter referred to as gene-22) is constructed by ligating a double-stranded DNA formed from a DNA having SEQ ID NO: 10 (hereinafter referred to as DNA-10) and a DNA having SEQ ID NO: 11 (hereinafter referred to as DNA-11), a double-stranded DNA formed from a DNA having SEQ ID NO: 12 (hereinafter referred to as DNA-12) and a DNA having SEQ ID NO: 13 (hereinafter referred to as DNA-13), a double-stranded DNA formed from a DNA having SEQ ID NO: 14 (hereinafter referred to as DNA-14) and a DNA having SEQ ID NO: 15 (hereinafter referred to as DNA-15) and a double-stranded DNA formed from a DNA having SEQ ID NO: 16 (hereinafter referred to as DNA-16) and a DNA having SEQ ID NO: 17 (hereinafter referred to as DNA-17) together using ligase.

Similarly, a plasmid containing a double-stranded DNA fragment represented by SEQ ID NO: 23 (hereinafter referred to as gene-23) is constructed using a double-stranded DNA formed from a DNA having SEQ ID NO: 18 (hereinafter referred to as DNA-18) and a DNA having SEQ ID NO: 19 (hereinafter referred to as DNA-19).

The double-stranded DNA fragments, gene-20, gene-21 and gene-22, prepared from the synthetic DNAs mentioned above have base sequences coding for the C-2 peptide, the C-14 peptide and the C-18 peptide, respectively. Gene-23 serves as a linker for joining each clone to the chum salmon growth hormone gene and contains a base sequence coding for methionine, glutamic acid and phenylalanine, which sequence is directly followed by a termination codon. The base sequence coding for glutamic acid and phenylalanine serves as an EcoRI recognition site.

[Step 2] Insertion of the linker gene into an sGH expression plasmid

The plasmid pSGH1B2 for sGH gene expression as produced by the method described in JP-A-61-93197 is used. A Bg1II-PvuI fragment (about 1.17 kb) of pSGH1B2, an NsiI-PvuI fragment (about 1.80 kb) of pSGH1B2, DNA-18 and DNA-19 were mixed and ligation of the double-stranded DNAs to each other was effected using DNA ligase to thereby construct a plasmid, pSGHE1, containing a gene coding for a polypeptide resulting from binding of glutamic acid and phenylalanine, via methionine, to a polypeptide comprising an aminoterminal methionine and the 1st to 103rd amino acids of sGH (cf. Fig. 1).

The amino acid sequence of the fused polypeptide expressed by pSGHE1, the DNA sequence coding for said polypeptide and the EcoRI site contained therein are shown under SEQ ID NO: 24. In SEQ ID NO: 24, the linker region is underlined.

[Step 3] Insertion of an HCV-associated antigen gene into sGH expression plasmids

The pSGHE1 constructed in step 2 is cleaved with EcoRI, then treated with bacterial alkaline phosphatase (BAP) and further cleaved with HindIII and PvuI, and an EcoRI-HindIII fragment of about 320 bp and an EcoRI-PvuI fragment of about 1.8 kb are isolated. Separately, pSGHE1 is cleaved with HindIII and PvuI, and a HindIII-PvuI fragment of about 850 bp is isolated. The above DNA fragments and the double-stranded DNA fragments formed from DNA-4 and DNA-5 are subjected to ligation using DNA ligase to give a plasmid named pSGHEC-2 (cf. Fig. 2). pSGHEC-2 holds the sGH gene of pSGHE1 and the gene coding for C-2 via a methionine-encoding gene. The amino acid sequence of the fused antigen polypeptide expressed by pSGHEC-2 and the DNA sequence coding for said polypeptide are shown under SEQ ID NO: 25. In SEQ ID NO: 25, the C-2 region is underlined.

The pSGHE1 constructed in step 2 is cleaved with EcoRI, then treated with bacterial alkaline phosphatase (BAP) and further cleaved with HindIII and PvuI, and an EcoRI-HindIII fragment of about 320 bp and an EcoRI-PvuI fragment of about 1.8 kb are isolated. Separately, after cleavage of pSGHE1 with HindIII and PvuI, a HindIII-PvuI fragment of about 850 bp is isolated. The DNA fragments mentioned above and the double-stranded DNA fragments formed from DNA-6 through DNA-9 are subjected to ligation using DNA ligase to give a plasmid named pSGHEC-14 (cf. Fig. 3). The pSGHEC-14 holds the sGH gene of pSGHE1 and the gene coding for C-14 via a methionine-encoding gene. The amino acid sequence of the fused antigen polypeptide expressed by pSGHEC-14 and the DNA sequence coding for said polypeptide are shown under SEQ ID NO: 26. In SEQ ID NO: 26, the C-14 region is underlined.

The pSGHE1 constructed in step 2 is cleaved with EcoRI, then treated with bacterial alkaline phosphatase (BAP) and further cleaved with HindIII and PvuI, and an EcoRI-HindIII fragment of about 320 bp and an EcoRI-PvuI fragment of about 1.8 kb are isolated. Separately, pSGHE1 is cleaved with HindIII and PvuI, and a HindIII-PvuI fragment of about 850 bp is isolated. The DNA fragments mentioned above and the double-stranded DNA fragments formed from DNA-10 through DNA-17 are subjected to ligation using DNA ligase to give a plasmid named pSGHEC-18 (cf. Fig. 4). The pSGHEC-18 holds the sGH gene of pSGHE1 and the gene coding for C-18 via a methionine-encoding gene. The amino acid sequence of the fused antigen polypeptide expressed by pSGHEC-18 and the DNA sequence coding for said polypeptide are shown under SEQ ID NO: 27. In SEQ ID NO: 27, the C-18 region is underlined.

[Step 4] Promoter substitution in pSGHEC-18

The pSGHEC-18 is cleaved with HindIII and PvuI and a DNA fragment of about 2.36 kb is isolated. The pKYP10 is cleaved with HindIII and PvuI and a DNA fragment of about 1.1 kb is isolated. The above DNAs are subjected to ligation using DNA ligase to give pSGHEC-18S (cf. Fig. 5). In pSGHEC-18S, the promoter of pSGHEC-18 has been substituted and pSGHEC-18S holds the gene coding for sGH and the gene coding for C-18 via a methionine-encoding gene. The amino acid sequence of the fused antigen polypeptide expressed by pSGHEC-18S and the DNA sequence coding for said polypeptide are shown under SEQ ID NO: 27. In SEQ ID NO: 27, the C-18 region is undelined.

[Step 5] Construction of expression vectors holding two or more HCV-associated antigen genes which may be the same or different

A typical example in which pSGHEC-14 constructed in step 3 is used is given. pSGHEC-14 is cleaved with PstI and then partially digested with the restriction enzyme EcoRI, and an EcoRI-PstI fragment of about 1.40 kb and an EcoRI-PstI fragment of about 1.85 kb are isolated. The above DNA fragments are subjected to ligation using DNA ligase to give a plasmid named pSGHEC-1414 (cf. Fig. 6). pSGHEC-1414 holds the sGH-encoding gene of pSGHE1 and a gene coding for two repetitions of C-14 via a methionine-encoding gene. The amino acid sequence of the fused antigen polypeptide expressed by pSGHEC-1414 and the DNA sequence coding therefor are shown under SEQ ID NO: 28. In SEQ ID NO: 28, the region for the two repetitions of C-14 is underlined. This step 5 makes it possible to link antigen genes of different kinds together and can be utilized in producing chimera antigens.

[Step 6] Production, isolation and purification of fused antigen polypeptides in which the chum salmon growth hormone is coupled to an HCV-associated antigen

The plasmid pSGHE1, pSGHEC-2, pSGHEC-14, pSGHEC-18S or pSGHEC-1414 produced in step 2, step 3, step 4 or step 5 is introduced into Escherichia coli. The Escherichia coli transformant obtained is cultured in a medium and the desired fused antigen polypeptide in which the chum salmon growth hormone is coupled to an HCV-associated antigen is recovered from the culture broth. The desired fused antigen polypeptide produced intracellularly by Escherichia coli occurs as water-insoluble granules. After cultivation, cells are collected and disrupted, the liquid obtained by cell disruption is centrifuged, and granules containing the fused antigen polypeptide in which the chum salmon growth hormone is coupled to the HCV-associated antigen are separated.

In the following, a method of purifying the fused antigen polypeptides is described. The fused antigen polypeptides produced in Escherichia coli transformants carrying the plasmids pSGHE1, pSGHEC-2, pSGHEC-14, pSGHEC-18S and pSGHEC-1414, respectively are hereinafter referred to as sGHE1, sGHEC-2, sGHEC-14, sGHEC-18S and sGHEC-1414, respectively.

[Step 7] Synthesis of HCV-associated antigen genes

First, DNAs having SEQ ID NO: 29, 30, 31, 32, 33, 34, 35 and 36 are chemically synthesized.

The synthesis of these DNAs is performed by the solid-phase phosphoamidite method using an automatic DNA synthesizer.

A double-stranded DNA formed from a DNA having SEQ ID NO: 29 (hereinafter referred to as DNA-29) and a DNA having SEQ ID NO: 30 (hereinafter referred to as DNA-30), a double-stranded DNA formed from a DNA having SEQ ID NO: 31 (hereinafter referred to as DNA-31) and a DNA having SEQ ID NO: 32 (hereinafter referred to as DNA-32) and a double-stranded DNA formed from a DNA having SEQ ID NO: 33 (hereinafter referred to as DNA-33) and a DNA having SEQ ID NO: 34 (hereinafter referred to as DNA-34) are subjected to ligation using ligase to construct a plasmid containing a double-stranded DNA fragment having SEQ ID NO: 37 (hereinafter referred to as gene-37).

Similarly, a plasmid containing a double-stranded DNA fragment having SEQ ID NO: 38 (hereinafter referred to as gene-38) is constructed by subjecting a double-stranded DNA formed from DNA-29 and DNA-30, a double-stranded DNA formed from DNA-31 and DNA-32 and a double-stranded DNA formed from a DNA having SEQ ID NO: 35 (hereinafter referred to as DNA-35) and a DNA having SEQ ID NO: 36 (hereinafter referred to as DNA-36) to ligation using ligase.

The double-stranded DNA fragments prepared from the synthetic DNAs as mentioned above, namely gene-37 and gene-38, have a base sequence coding for the C-14 peptide and a base sequence coding for a peptide derived from the C-14 peptide by deletion of the 32nd to 36th amino acids, respectively. In gene-37, the base sequence has been partly modified without changing the amino acid sequence of C-14; thus, in the base sequence for C-14, the 15th base A has been replaced by G, the 33rd base A by G, the 66th C by T, the 69th C by T, and the 99th base A by G. Gene-38 is a synthetic DNA corresponding to gene-37 but missing the 93rd to the 107th bases of the base sequence thereof.

Hereinafter, the peptide encoded by gene-37 or the DNA coding for said peptide is referred to as C-14A, and the peptide encoded by gene-38 or the DNA coding for said peptide as C-14B.

[Step 8] Insertion of HCV-associated antigen genes into sGH expression plasmids

pSGHE1 constructed in step 2 is cleaved with EcoRI, then treated with bacterial alkaline phosphatase (BAP) and further cleaved with HindIII and PvuI, and an EcoRI-HindIII fragment of about 320 bp and an EcoRI-PvuI fragment of about 1.8 kb are isolated. Separately, pSGHE1 is cleaved with HindIII and PvuI, and a HindIII-PvuI fragment of about 850 bp is isolated. The DNA fragments mentioned above and double-stranded DNA fragments formed from DNA-29 to DNA-34 are subjected to ligation using DNA ligase to give a plasmid named pSGHEC-14A (cf. Fig. 8). pSGHEC-14A holds the sGH gene of pSGHE1 and the C-14A-encoding gene via a methionine-encoding gene. The amino acid sequence of the fused antigen polypeptide expressed by pSGHEC-14A and the DNA sequence coding for said polypeptide are shown under SEQ ID NO: 39. In SEQ ID NO: 39, the C-14A region is underlined.

pSGHE1 constructed in step 2 is cleaved with EcoRI, then treated with bacterial alkaline phosphatase (BAP) and further cleaved with HindIII and PvuI, and an EcoRI-HindIII fragment of about 320 bp and an EcoRI-PvuI fragment of about 1.8 kb are isolated. Separately, pSGHE1 is cleaved with HindIII and PvuI, and a HindIII-PvuI fragment of about 850 bp is isolated. The DNA fragments mentioned above and double-stranded DNA fragments formed from DNA-29 to DNA-32 and DNA-35 to DNA-36 are subjected to ligation using DNA ligase to give a plasmid named pSGHEC-14B (cf. Fig. 9). pSGHEC-14B holds the sGH gene of pSGHE1 and the C-14B-encoding gene via a methionine-encoding gene. The amino acid sequence of the fused antigen polypeptide expressed by pSGHEC-14B and the DNA sequence coding for said polypeptide are shown under SEQ ID NO: 40. In SEQ ID NO: 40, the C-14B region is underlined.

[Step 9] Production, isolation and purification of fused antigen polypeptides in which the chum salmon growth hormone is coupled to an HCV-associated antigen (C-14A or C-14B)

Using the plasmids pSGHEC-14A and pSGHEC-14B produced in step (8) and following the procedure described in step 6, fused antigen polypeptides are produced, and isolated and purified. The fused antigen polypeptides encoded by the plasmids pSGHEC-14A and pSGHEC-14B are referred to as sGHEC-14A and sGHEC-14B, respectively.

Purification of fused antigen polypeptides

(1) Preparation of roughly purified fused antigen polypeptide fractions

The roughly purified fractions can be prepared by an appropriate combination of known methods, preferably in the following manner.

When the fused antigen polypeptide is produced in the form of granules, the granules are collected by the method described in JP-A-60-244259 and dissolved in a buffer (pH 5 to 12) containing a denaturating agent (preferably 4 M or a higher concentration of urea, or 3 M or a higher concentration of guanidine hydrochloride) or in a solubilizing agent solution (preferably 50% or more concentrated formic acid). The supernatant is purified as necessary in the presence of the denaturating or solubilizing agent. Then, the denaturating agent or solubilizing agent is removed to give a roughly purified fraction. Such purification in the presence of a denaturating or solubilizing agent and removal of the denaturating or solublizing agent can be performed using an appropriate combination of known methods [JP-A-1-131195; Japanese Biochemical Society (ed.): Seikagaku Jikken Koza (Experiments in Biochemistry) 1, Tanpakushitsu no Kagaku (Chemistry of Proteins) II, published by Tokyo Kagaku Dozin, pp. 11-354 (1976); Japanese Biochemical Society (ed.): Seikagaku Jikken Koza, second series, 2, Tanpakushitsu no Kagaku, Book 1, published by Tokyo Kagaku Dozin, pp. 3-12 and pp. 8-186 (1987)].

When the fused antigen polypeptide is produced in an intracellular soluble fraction, cells are disrupted by the method described in JP-A-63-17898, and the supernatant is separated as a roughly purified fraction. When the fused protein is produced in the culture supernatant, the culture supernatant is separated by centrifugation and used as a roughly purified fraction.

(2) Purification of fused antigen polypeptides

The fused antigen polypeptide can be purified from the roughly purified fraction using an appropriate combination of known methods [Japanese Biochemical Society (ed.): Seikagaku Jikken Koza 1, Tanpakushitsu no Kagaku II, published by Tokyo Kagaku Dozin, pp. 11-354 and pp. 81-186 (1987)].

The present invention also provides a method of detecting an anti-HCV antibody using the above-mentioned fused antigen polypeptide.

In the following, the anti-HCV antibody detecting method using the fused antigen polypeptide is described.

In the practice of the invention, the following immunological methods may be used as described in the respective references:

- o Enzyme immunoassay (hereinafter referred to as EIA)
  Eiji Ishikawa et al.: Koso Men'eki Sokuteiho (Enzyme Immunoassay), published by Igaku Shoin, 1978.
- o Radioimmunoassay (hereinafter referred to as RIA)
  Shinya Inai et al.: Men'eki Kesseigaku (Immuno serology), published by Ishiyaku Shuppan, 1981.
- o Fluorescence immunoassay (hereinafter referred to as FIA)
  Vide supra.
- o Reverse passive agglutination
  Vide supra
- o Phytohematoagglutination
  Vide supra.

- o Laser nephrometry
  Vide supra.
- o Immune hemolysis (using erythrocytes or liposomes)
  Vide supra.
- o Particle agglutination
  Saishin Kensa, published by Itensha, Vol. 2, No. 2, pp. 151-157, 1984; Gann, 75, 845, 1984.
- o Western blotting

In RIA, EIA and FIA, not only homogenous systems but also heterogenous systems, for example in the sandwich technique, can be analyzed.

A typical exmaple in which EIA is performed by the sandwich technique is mentioned below.

Wells of a microtiter plate are coated with the fused antigen polypeptide. A serum sample diluted with an appropriate buffer, for example buffer A [1/15 M sodium phosphate buffer (pH 7.2) containing 0.15 M NaCl, 0.1% gelatin, 1% bovine serum albumin and 0.1% $NaN_3$], is placed in the wells. The plate is allowed to stand at 4 to 37°C for 1 hour to 1 week and then the wells are washed thoroughly with an appropriate buffer, for example buffer B [1/15 M sodium phosphate buffer (pH 7.2) containing 0.05% Tween 20 and 0.15 M NaCl].

A conjugate prepared by binding a rabbit anti-human immunoglobulin antibody Fab fragment to horseraddish peroxidase by the periodic acid method is diluted with an appropriate buffer, for example buffer B, and the dilution is placed in the wells. After 1 to 24 hours of standing at 4 to 45°C, the plate is washed with the same buffer. Then, an appropriate substrate solution, for example an orthophenylenediamine substrate solution [1/15 M sodium phosphate buffer (pH 7.2) containing 0.02% orthophenylenediamine and 35 mM hydrogen peroxide] is distributed into the wells, and the plate is allowed to stand at 15 to 37°C for 5 to 60 minutes. The reaction is terminated with 2 N $H_2SO_4$ and, after 1 to 10 minutes of standing at room temperature, optical density (O.D.) measurements are carried out at 410 nm and 600 nm using a photometer for microtiter plates. The value obtained by subtracting the latter value from the former value is taken as the antibody titer.

The procedure mentioned above is nothing but an example. Other antibodies, substrate solutions and color formers may be used where appropriate and various modifications may be made.

An example of anti-HCV antibody assay carried out in the manner of Western blotting is given below.

A purified fused antigen polypeptide is directly dissolved in Laemmli's sample buffer with heating and electrophoresed on an SDS-polyacrylamide gel. The protein on said gel is transferred to a nitrocellulose membrane and Western blotting [Burnett: Anal. Biochem., 112, 195 (1981)] is performed using a normal human serum and the serum from a hepatitis C patient.

The reaction conditions in the above-mentioned recombinant DNA technology are generally as follows:

DNA digestion with a restriction enzyme or enzymes is generally carried out in a reaction mixture containing 0.1 to 20 µg of DNA, 2 to 200 mM (preferably 10 to 40 mM) Tris-HCl (pH 6.0 to 9.5, preferably 7.0 to 8.0), 0 to 200 mM NaCl or KCl, 2 to 30 mM (preferably 5 to 10 mM) $MgCl_2$ and 0 to 20 mM 2-mercaptoethanol at 20 to 70°C (the optimum temperature varies depending on the restriction enzyme) for 15 minutes to 24 hours using 0.1 to 100 units (preferably 1 to 3 units per µg of DNA) of each restriction enzyme.

DNA fragments formed upon restriction enzyme digestion are purified by the LGT method or by polyacrylamide gel electrophoresis, for instance.

Ligation of DNA fragments is carried out in a reaction mixture containing 2 to 200 mM (preferably 10 to 40 mM) Tris-HCl (pH 6.1 to 9.5, preferably 7.0 to 8.0), 2 to 20 mM (preferably 5 to 10 mM) $MgCl_2$, 0.1 to 10 mM (preferably 0.5 to 2.0 mM) ATP and 1 to 50 mM (preferably 5 to 10 mM) dithiothreitol at 1 to 37°C (preferably 3 to 20°C) for 15 minutes to 72 hours (preferably 2 to 20 hours) using 0.3 to 10 units of T4 DNA ligase.

The recombinant plasmid DNA formed by the ligation reaction is introduced into Escherichia coli as necessary by the transformation method of Cohen et al. [S. N. Cohen et al.: Proc. Natl. Acad. Sci. U.S.A., 69, 2110 (1972)].

The recombinant plasmid DNA can be isolated from Escherichia coli carrying said DNA, for exmaple by cesium chloride-ethidium bromide density gradient ultracentrifugation [D. B. Clewell et al.: Proc. Natl. Acad. Sci. U.S.A., 62, 1159 (1969)] or the method of Birnboim et al. [H. C. Birnboim et al.: Nucleic Acids Res., 7, 1513 (1979)].

The plasmid DNA is digested with a restriction enzyme or enzymes and the cleavage site or sites thereof are examined by agarose gel electrophoresis or polyacrylamide gel electrophoresis. Furthermore, where DNA base sequence determination is necessary, the Maxam-Gilbert method [Proc. Natl. Acad. Sci. U.S.A., 74, 560 (1977)] or the Sanger method using M13 phage [Sanger et al.: Proc. Natl. Acad. Sci. U.S.A.,

74, 5463 (1977); Amersham's M13 Cloning and Sequencing Handbook] can be used.

The fused antigen polypeptides of this invention can be produced in the following manner.

Thus, Escherichia coli W3110 strA or HB101, for instance, is transformed with the plasmid and a colony of Escherichia coli carrying the plasmid is selected from among ampicillin-resistant colonies. The Escherichia coli transformant carrying the plasmid is cultivated in a medium, whereby the fused antigen polypeptide is produced in the culture broth.

The medium to be used may be a synthetic one or a natural one provided that it is suited for the growth of Escherichia coli and the production of the fused antigen polypeptide.

Glucose, fructose, lactose, glycerol, mannitol and sorbitol, for instance, can be used as carbon sources, and $NH_4Cl$, $(NH_4)_2SO_4$, casamino acids, yeast extract, polypeptone, meat extract, Bacto-tryptone, corn steep liquor and the like as nitrogen sources. $K_2HPO_4$, $KH_2PO_4$, NaCl, $MgSO_4$, vitamin $B_1$, $MgCl_2$ and so forth can be used as other nutrient sources.

The cultivation is carried out at a pH of 5.5 to 8.5 and a temperature of 18 to 40 ° C with aeration and stirring.

After 5 to 90 hours of cultivation, the fused antigen polypeptide is found accumulated in cultured cells as water-insoluble granules. Cells are harvested from the culture and disrupted. The dusruption product is subjected to centrifugation and the fused antigen polypeptide is isolated from the sediment by gel filtration column chromatography or HPLC, for instance.

Said fused antigen polypeptide can be detected by heating cultured cells directly in Laemmli's sample buffer [Laemmli: Nature, 227, 680 (1970)] for dissolution, applying the solution to an SDS-polyacrylamide gel [method of Laemmli: vide supra.], followed by staining with Coomassie Brilliant Blue (Bio-Rad).

EXAMPLES

Example 1: Production of DNA-4 to DNA-19

DNA-4 through DNA-19 were synthesized by the solid-phase phosphoamidite method [S. L. Beaucage et al.: Tetrahedron Letters, 22, 1859 (1981); L. J. McBrie et al.: ibid., 24, 245 (1983)] using an Applied Biosystems model 380A automatic DNA synthesizer, as follows:

Silica gel was used as the solid carrier. (1) A nucleotide was condensed, by the phosphoamidite method, with the 5' hydroxy group of a nucleotide bound to said carrier via its 3' hydroxy group, (2) the phosphite bond of the condensed nucleotide is converted to a phosphate bond by oxidation with iodine and (3) the protective group on the 5' hydroxy group of the condensed nucleotide was removed using trifluoroacetic acid. Then, step (1) was repeated for condensing a next nucleotide in the same manner. Steps (1) to (3) were thus repeated and a DNA was synthesized on the carrier. After completion of the synthesis, the carrier-bound DNA was allowed to stand in thiophenol solution at room temperature for 1 hour for eliminating the protective group on the phosphoric acid moiety and then allowed to stand in concentrated aqueous ammonia at room temperature for 1 hour, whereby the DNA was released from the carrier. The DNA-containing concentrated aqueous ammonia was heated in a sealed tube at 60 ° C for 12 hours for eliminating the protective groups on the bases.

In the case of DNA-4, for instance, the synthesis was carried out using the carrier with 0.2 micromole of the nucleotide bound thereon and, the reactions for elimination of protective groups and release from the solid phase were carried out to provide 21 O.D. units (measured at 260 nm) of crude DNA-4. Five O.D. units of this crude product was purified by 10% polyacrylamide gel (2 mm thick, 13 cm x 13 cm) electrophoresis using Tris-borate buffer (pH 8) containing 7 M urea. The gel region containing DNA-4 was collected and subjected to 18 hours of extraction with 1 ml of 0.2 M triethylamine carbonate buffer (pH 8) (hereinafter referred to as TEAB). The extract was then applied to a Sephadex DE52 column (6 mm in diameter, 5 mm long) for adsorption of DNA-4. Elution with 2 ml of 2 M TEAB gave 1.4 O.D. units of pure DNA-4.

Other DNAs than DNA-4 were synthesized in like yields.

These DNAs were radiolabeled by the conventional phosphorylation of the 5' hydroxy group using phage T4 polynucleotide kinase and [$\gamma$-$^{32}$P]ATP [A. M. Maxam et al.: Methods in Enzymology, Vol. 65, Part 1, p. 499, Academic Press (1980)]. The labeled DNAs were subjected to 12% polyacrylamide gel electrophoresis using Tris-borate buffer containing 7 M urea, whereby the purity and chain length of each DNA were confirmed.

Example 2: Isolation and purification of the plasmids pKYP10 and pSGH1B2

A pKYP10-carrying Escherichia coli strain [Escherichia coli HB101; Bolivar et al.: Gene, 2, 75 (1977)] and a pSGH1B2-carrying Escherichia coli strain [Escherichia coli HB101] (JP-A-61-93197) were respectively cultured in 10 ml of L medium (1% Bacto-tryptone, 0.5% yeast extract, 1% NaCl, pH 7.5) containing 50 µg/ml of ampicillin at 37° C for 18 hours.

The whole volume of each culture was transferred to 1 liter of L medium containing 50 µg/ml ampicillin and cultivation was performed at 37° C. After 4 hours, chloramphenicol was added at a final concentration of 170 µg/ml, and cultivation was further conducted at 37° C for 16 hours. Cells were harvested by centrifugation (5,000 rpm, 10 minutes), washed with 0.8% NaCl, and suspended in 20 ml of 50 mM Tris-HCl (pH 8.0). After the suspension was ice-cooled, 8 ml of 10 mg/ml lysozyme was added, and the mixture was allowed to stand on ice for 10 minutes. Then, 9.6 ml of 0.5 M EDTA was added and the mixture was allowed to stand on ice for 10 minutes. Following further addition of 2.3 ml of 2% Triton X-100 (Wako Pure Chemical Industries), the mixture was further allowed to stand on ice for 1 hour. Ultracentrifugation was performed at 50,000 x g and 4° C for 1 hour. The thus-obtained supernatant (about 40 ml) was adjusted to pH 12.5 with 3 M NaOH and gently stirred at room temperature for 10 minutes.

The supernatant was then adjusted to pH 8.5 by addition of 2 M Tris-HCl (pH 7.5) and further stirred for 3 minutes. At this point of time, the liquid had a volume of about 55 ml. After addition of 1/9 volume of 5 M NaCl, equal volumes of 10 mM Tris-HCl (pH 7.5) and phenol saturated with 1 mM EDTA were added, and the resultant mixture was stirred vigorously, and the aqueous layer was collected by low-speed centrifugation (3,300 rpm, 10 minutes; hereinafter the same conditions were used) (hereinafter this treatment is briefly referred to as phenol extraction). To said layer was added 1/250 vlume of 5 mg/ml RNase A (Sigma), and RNA decomposition was carried out at 37° C for 1 hour. Then, 1/5 volume of 5 M NaCl was added, then 1/3 volume of 30% PEG 6000 (Nakalai Tesque) was added, and the mixture was allowed to stand at -20° C for 2 hours. The precipitate was collected by centrifugation and dissolved in 2 ml of a solution containing 10 mM Tris-HCl (pH 7.5) and 1 mM EDTA. After sodium dodecyl sulfate (SDS) was added at a final concentration of 0.5%, proteinase K (Sigma) was added at a final concentration of 50 µg/ml and proteolysis was effected at 37° C for 1 hour.

After three repetitions of phenol extraction, the reaction mixture was stirred vigorously with an equal volume of chloroform and then the aqueous layer was collected by low-speed centrifugation. Following addition of 1/10 volume of 3 M sodium acetate, 2.5 volumes of ethanol was added, and the mixture was allowed to stand at -20° C for 1 hour. The precipitate was collected by centrifugation under cooling (4° C, 11,000 rpm, 10 minutes) and dissolved in 1 ml of a liquid comprising 10 mM Tris-HCl (pH 7.5) and 1 mM EDTA. In this manner, 800 µg each of pKYP10 and pSGH1B2 could be obtained. The structure of pKYP10 was verified by cleavage with EcoRI, KpnI, BamHI, BglII and PstI (Takara Shuzo) followed by agarose gel electrophoresis. The structure of pSGH1B2 was confirmed by cleavage with EcoRI, HindIII, BglII, BamHI, NsiI and PstI (Takara Shuzo) followed by agarose gel electrophoresis.

Example 3: Construction of the plasmid pSGHE1

Two micrograms of the plasmid pSGH1B2 obtained in Exmaple 2 was dissolved in 30 µl of a solution [10 mM Tris-HCl (pH 7.5), 7 mM $MgCl_2$, 6 mM 2-mercaptoethanol, 100 mM Nacl] containing 10 units of the restriction enzyme PvuI (Takara Shuzo), 10 units of BglII (Takara Shuzo) and 10 units of NsiI (Takara Shuzo), and the digestion reaction was carried out at 37° C for 2 hours. This reaction mixture was subjected to electrophoresis on an agarose gel containing ethidium bromide, and a gel segment containing a DNA of about 1.17 kb and a gel segment containing a DNA of about 1.80 kb were excised following detection with ultraviolet light (wavelength 302 nm). To each gel section was added 0.5 ml of phenol, followed by freezing and thawing. The aqueous layer was washed with chloroform and the DNA was then recovered by ethanol precipitation.

DNA-18 and DNA-19 (10 picomoles each) were respectively dissolved in 30 µl of T4 polynucleotide kinase reaction buffer [50 mM Tris-HCl (pH 7.5), 10 mM $MgCl_2$, 5 mM dithiothreitol (hereinafter abbreviated as DTT), 1 mM ATP, 0.1 mM spermidine, 0.1 mM EDTA], 3 units of T4 polynucleotide kinase (Takara Shuzo) was added, and the phosphorylation reaction was conducted at 37° C for 40 minutes. The enzyme was then deactivated by heating at 65° C for 15 minutes. The reaction mixtures (4 µl each) were combined, the DNA fragments prepared as described above (0.08 picomole each) were added, and the mixture was adjusted to the composition of T4 ligase reaction buffer [28 mM Tris-HCl (pH 7.5), 9 mM $MgCl_2$, 10 mM DTT, 0.03 mM EDTA, 0.7 mM ATP, 0.03 mM spermidine]. Then, 175 units of T4 DNA ligase (Takara Shuzo) was added. The volume was 30 µl. The ligation reaction was then carried out at 4° C for 16 hours.

Using this reaction mixture, Escherichia coli HB101 [Bolivar et al.: Gene, 2, 75 (1977)] was transformed by the method of Cohen et al. [S. N. Cohen et al.: Proc. Natl. Acad. Sci. U.S.A., 69, 2110 (1972)] to give ampicillin-resistant ($Ap^r$) colonies. The plasmid DNA was recovered from one of the colonies by the alkaline treatment method [Maniatis et al. (ed.): Molecular Cloning, published by Cold Spring Harbor Laboratory, page 368]. pSGHE1 was thus obtained. The structure of pSGHE1 was confirmed by agarose gel electrophoresis following cleavage with BglII, PvuI, HindIII, EcoRI and NsiI. The restriction enzyme cleavage reaction was carried out in a solution containing 10 mM Tris-HCl (pH 7.5), 7 mM $MgCl_2$ and 6 mM 2-mercaptoethanol with NaCl added thereto to an optimum concentration (0 to 200 mM) for the enzyme used.

Example 4: Construction of the plasmid pSGHEC-2

The plasmid pSGHE1 (2 μg) obtained in Example 3 was dissolved in 30 μl of the restriction enzyme reaction buffer (100 mM NaCl) mentioned in Example 3, 10 units of EcoRI (Takara Shuzo) was added, and digestion was effected at 37°C for 2 hours. To this reaction mixture was then added 1 unit of bacterial alkaline phosphatase (Takara Shuzo), and the reaction was carried out at 65°C for 30 minutes. Then, 10 units of HindIII (Takara Shuzo) and 10 units of PvuI (Takara Shuzo) were added, and the digestion reaction was conducted at 37°C for 2 hours. In parallel, pSGHE1 was digested directly with 10 units of HindIII (Takara Shuzo) and 10 units of PvuI (Takara Shuzo) at 37°C for 2 hours.

The above reaction mixtures were subjected to electrophoresis on an agarose gel containing ethidium bromide and, using ultraviolet light (302 nm in wavelength) for detection, gel portions containing DNA fragments of about 320 bp, about 850 bp and about 1.8 kb, respectively, were excised. Phenol (0.5 ml) was added to each gel section, the whole was frozen and thawed, and the aqueous layer was washed with chloroform. Each DNA fragment mentioned above was recovered by ethanol precipitation.

DNA-4 and DNA-5 (10 picomoles each) were respectively dissolved in 30 μl of T4 polynucleotide kinase reaction buffer [50 mM Tris-HCl (pH 7.5), 10 mM $MgCl_2$, 5 mM DTT, 1 mM ATP, 0.1 mM spermidine, 0.1 mM EDTA] and, after addition of 3 units of T4 polynucleotide kinase (Takara Shuzo) to each solution, the phosphorylation reaction was carried out at 37°C for 40 minutes. The enzyme was then deactivated by heating at 65°C for 15 minutes. The reaction mixtures (4 μl each) were combined, the DNA fragments obtained as described above (0.08 picomole each) were added, the mixture was adjusted to the composition of T4 ligase reaction buffer [28 mM Tris-HCl (pH 7.5), 9 mM $MgCl_2$, 10 mM DTT, 0.03 mM EDTA, 0.7 mM ATP, 0.03 mM spermidine], and 175 units of T4 DNA ligase (Takara Shuzo) was added. The volume was 30 μl. The ligation was then carried out at 4°C for 16 hours.

Using this reaction mixture, Escherichia coli HB101 was transformed by the method of Cohen et al. to give ampicillin-resistant ($Ap^r$) colonies. Plasmid DNA recovery from one of these colonies by the alkaline treatment method gave pSGHEC-2. The structure of pSGHEC-2 was confirmed by cleavage with BglII, PvuI, HindIII and EcoRI followed by agarose gel electrophoresis. The restriction enzyme cleavage reaction was carried out in a solution containing 10 mM Tris-HCl (pH 7.5), 7 mM $MgCl_2$ and 6 mM 2-mercaptoethanol with NaCl added to an optimum concentration (0 to 200 mM) for the enzyme used.

The Escherichia coli strain carrying pSGHEC-2 has been deposited with the Fermentation Research Institute (FRI), Agency of Industrial Science and Technology as of January 18, 1990 under the Budapest Treaty under the designation and deposit number of Escherichia coli SGHEC2, FERM BP-2732.

Example 5: Construction of the plasmid pSGHEC-14

The plasmid pSGHE1 (2 μg) obtained in Example 3 was dissolved in 30 μl of the restriction enzyme reaction buffer (100 mM NaCl) mentioned in Example 2, then 10 units of EcoRI (Takara Shuzo) was added, and digestion was effected at 37°C for 2 hours. To this reaction mixture was then added 1 unit of bacterial alkaline phosphatase (Takara Shuzo), and the mixture was incubated at 65°C for 30 minutes. Then, 10 units of HindIII (Takara Shuzo) and 10 units of PvuI (Takara Shuzo) were added, and digestion was effected at 37°C for 2 hours. Separately, pSGHE1 was digested directly with 10 units of HindIII (Takara Shuzo) and 10 units of PvuI (Takara Shuzo) at 37°C for 2 hours.

These reaction mixtures were subjected to electrophoresis on an agarose gel containing ethidium bromide and, using ultraviolet light (302 nm in wavelength) for detection, gel segments containing DNA fragments of about 320 bp, about 850 bp and about 1.8 kb were excised. Phenol (0.5 ml) was added to each gel section, followed by freezing and thawing. The aqueous layer was then washed with chloroform and each DNA fragment mentioned above was recovered by ethanol precipitation.

DNA-6 through DNA-9 (10 picomoles each) were respectively dissolved in 30 μl of T4 polynucleotide kinase reaction buffer [50 mM Tris-HCl (pH 7.5), 10 mM $MgCl_2$, 5 mM DTT, 1 mM ATP, 0.1 mM spermidine, 0.1 mM EDTA], then 3 units of T4 polynucleotide kinase (Takara Shuzo) was added to each solution, and the phosphorylation reaction was carried out at 37°C for 40 minutes. The enzyme was then inactivated by heating at 65°C for 15 minutes. These reaction mixtures (4 μl each) were combined and the DNA fragments obtained as described above (0.08 picomole each) were added. The resulting mixture was adjusted to the composition of T4 ligase reaction buffer [28 mM Tris-HCl (pH 7.5), 9 mM $MgCl_2$, 10 mM DTT, 0.03 mM EDTA, 0.7 mM ATP, 0.03 mM spermidine] and 175 units of T4 DNA ligase (Takara Shuzo) was added. The final volume was 30 µl. Then, ligation was effected at 4°C for 16 hours.

Using this reaction mixture, Escherichia coli HB101 was transformed by the method of Cohen et al. to give ampicillin-resistant ($Ap^r$) colonies. Plasmid DNA recovery from one of these colonies by the alkaline treatment method gave pSGHEC-14. The structure of pSGHEC-14 was confirmed by cleavage with BglII, PvuI, HindIII and EcoRI followed by agarose gel electrophoresis. The restriction enzyme cleavage reaction was carried out in a solution containing 10 mM Tris-HCl (pH 7.5), 7 mM $MgCl_2$ and 6 mM 2-mercaptoethanol with NaCl added to an optimum concentration (0 to 200 mM) for the enzyme used.

The Escherichia coli transformant carrying pSGHEC-14 has been deposited with the Fermentation Research Institute (FRI), Agency of Industrial Science and Technology as of January 18, 1990 under the Budapest Treaty under the designation and deposit number of Escherichia coli SGHEC14 and FERM BP-2731, respectively.

Example 6: Construction of the plasmid pSGHEC-18

The plasmid pSGHE1 (2 µg) obtained in Example 3 was dissolved in 30 µl of the restriction enzyme reaction buffer (100 mM NaCl) mentioned in Example 2, 10 units of EcoRI (Takara Shuzo) was added, and the digestion reaction was carried out at 37°C for 2 hours. To this reaction mixture was added 1 unit of bacterial alkaline phosphatase (Takara Shuzo), and the reaction was carried out at 65°C for 30 minutes. Then, 10 units of HindIII (Takara Shuzo) and 10 units of PvuI (Takara Shuzo) were added, and the digestion reaction was conducted at 37°C for 2 hours. Separately, pSGHE1 was digested with 10 units of HindIII (Takara Shuzo) and 10 units of PvuI (Takara Shuzo) at 37°C for 2 hours.

These reaction mixtures were subjected to electrophoresis on an agarose gel containing ethidium bromide and, using ultraviolet light (302 nm in wavelength) for detection, gel portions containing DNA fragments of about 320 bp, about 850 bp and about 1.8 kb were excised. Phenol (0.5 ml) was added to each gel section and, after freezing and thawing, the aqueous layer was separated and washed with chloroform. The DNA fragments mentioned above were then recovered by ethanol precipitation.

DNA-10 through DNA-17 (10 picomoles each) were respectively dissolved in 30 µl of T4 polynucleotide kinase reaction buffer [50 mM Tris-HCl (pH 7.5), 10 mM $MgCl_2$, 5 mM DTT, 1 mM ATP, 0.1 mM spermidine, 0.1 mM EDTA], then 3 units of T4 polynucleotide kinase (Takara Shuzo) was added to each solution, and the phosphorylation reaction was conducted at 37°C for 40 minutes. The enzyme was then deactivated by heating at 65°C for 15 minutes. The reaction mixtures (4 µl each) were combined, the DNA fragments obtained as described above (0.08 picomole each) were added, the mixture was adjusted to the composition of T4 ligase reaction buffer [28 mM Tris-HCl (pH 7.5), 9 mM $MgCl_2$, 10 mM DTT, 0.03 mM EDTA, 0.7 mM ATP, 0.03 mM spermidine] and 175 units of T4 DNA ligase (Takara Shuzo) was added. The final volume was 30 µl. Ligation was then carried out at 4°C for 16 hours.

Using this reaction mixture, Escherichia coli HB101 was transformed by the method of Cohen et al. to give ampicillin-resistant ($Ap^r$) colonies. Plasmid DNA recovery from one of these colonies by the alkaline treatment method gave pSGHEC-18. The structure of pSGHEC-18 was confirmed by cleavage with BglII, PvuI, HindIII an EcoRI followed by agarose gel electrophoresis. The restriction enzyme cleavage reaction was carried out in a solution containing 10 mM Tris-HCl (pH 7.5), 7 mM $MgCl_2$ and 6 mM 2-mercaptoethanol with NaCl added to an optimum concentration (0 to 200 mM) for the enzyme used.

Emample 7: Construction of the plasmid pSGHEC-18S

The plasmid pSGHEC-18 (2 µg) obtained in Example 6 and 2 µg of pKYP10 (JP-A-58-110600) were respectively dissolved in 30 µl of a solution [10 mM Tris-HCl (pH 7.5), 7 mM $MgCl_2$, 6 mM 2-mercaptoethanol, 100 mM NaCl] containing 10 units of HindIII (Takara Shuzo) and 10 units of PvuI (Takara Shuzo), and the digestion reaction was carried out at 37°C for 2 hours. The reaction mixtures were subjected to electrophoresis on an agarose gel containing ethidium bromide and, using ultraviolet light (wavelength 302 nm) for detection, gel portions containing a pSGHEC-18 derived DNA fragment of about 2.36 kb and a pKYP10-derived DNA fragment of about 1.1 kb, respectively, were excised. Phenol (0.5 ml) was added to each gel section and, after freezing and thawing, the aqueous layer was separated and washed with chloroform. The DNA fragments mentioned above were then recovered by ethanol precipitation.

The DNA fragments obtained as described above (0.08 picomole each) were combined, the mixture was adjusted to the composition of T4 ligase reaction buffer [28 mM Tris-HCl (pH 7.5), 9 mM $MgCl_2$, 10 mM DDT, 0.03 mM EDTA, 0.7 mM ATP, 0.03 mM spermidine] and 175 units of T4 DNA ligase (Takara Shuzo) was added. The final volume was 30 µl. The ligation reaction was then carried out at 4°C for 16 hours.

Using this reaction mixture, Escherichia coli HB101 was transformed by the method of Cohen et al. to give ampicillin-resistant ($Ap^r$) colonies. Plasmid DNA recovery from one of these colonies by the alkaline treatment method gave pSGHEC-18S. The structure of pSGHEC-18S was confirmed by cleavage with BglII, PvuI, HindIII and EcoRI followed by agarose gel electrophoresis. The reaction enzyme cleavage reaction was conducted in a solution containing 10 mM Tris-HCl (pH 7.5), 7 mM $MgCl_2$ and 6 mM 2-mercaptoethanol with NaCl added to an optimum concentration (0 to 200 mM) for the enzyme used.

The Escherichia coli transformant carrying pSGHEC-18S has been deposited with the Fermentation Research Institute (FRI), Agency of Industrial Science and Technology as of January 18, 1990 under the Budapest Treaty under the designation and deposit number of Escherichia coli SGHEC18S and FERM BP-2729, respectively.

Example 8: Construction of the plasmid pSGHEC-1414

The plasmid pSGHEC-14 (5 µg) obtained in Example 5 was dissolved in 30 µl of the restriction enzyme reaction buffer (100 mM NaCl) mentioned in Example 3, 10 units of PstI (Takara Shuzo) was added, and the digestion reaction was carried out at 37°C for 2 hour. Then, 1 units of EcoRI (Takara Shuzo) was added to that reaction mixture and partial digestion was effected at 37°C for 1 hour. This reaction mixture was subjected to electrophoresis on an agarose gel containing ethidium bromide and, using ultraviolet light (wavelength 302 nm) for detection, gel portions containing DNA fragments of about 1.4 kb and about 1.85 kb, respectively, were excised. Phenol (0.5 ml) was added to each gel section and, after freezing and thawing, the aqueous layer was washed with chloroform. The DNA fragments mentioned above were then recovered by ethanol precipitation.

The DNA fragments (0.08 picomole each) obtained as described above were combined to make 10 ml, the mixture was adjusted to the composition of T4 ligase reaction buffer [28 mM Tris-HCl (pH 7.5), 9 mM $MgCl_2$, 10 mM DTT, 0.03 mM EDTA, 0.7 mM ATP, 0.03 mM spermidine] and 175 units of T4 DNA ligase (Takara Shuzo) was added. The final volume was 30 µl. The ligation reaction was then carried out at 4°C for 16 hours.

Using this reaction mixture, Escherichia coli HB101 was transformed by the method of Cohen et al. to give ampicillin-resistant ($Ap^r$) colonies. Plasmid DNA recovery from one of these colonies by the alkaline treatment method gave pSGHEC-1414. The structure of pSGHEC-1414 was confirmed by cleavage with BglII, PvuI, HindIII and EcoRI followed by agarose gel electrophoresis. The restriction enzyme cleavage reaction was conducted in a solution containing 10 mM Tris-HCl (pH 7.5), 7 mM $MgCl_2$ and 6 mM 2-mercaptoethanol with NaCl added to an optimum concentration (0 to 200 mM) for the enzyme used.

The Escherichia coli transformant carrying pSGHEC-1414 has been deposited with the Fermentation Research Institute (FRI), Agency of Industrial Science and Technology as of January 18, 1990 under the Budapest Treaty under the designation and deposit number of Escherichia coli SGHEC1414 and FERM BP-2730, respectively.

Example 9: Production of sGHE1 in Escherichia coli with the plasmid pSGHE1 introduced thereinto

Using pSGHE1 obtained in Example 3, Escherichia coli W3110 strA (FERM BP-732) was transformed by the method described in Example 3. An $Ap^r$ colony obtained was inoculated into 8 ml of LG medium (1% Bacto-tryptone, 0.5% yeast extract, 0.5% NaCl, 0.1% glucose, 50 µg/ml ampicillin, pH 7.4) and cultured at 30°C for 16 hours. A 200 µl portion of this culture was inoculated into 10 ml of MCG medium (0.6% $Na_2HPO_4$,0.3% $KH_2PO_4$, 0.05% NaCl, 0.1% $NH_4Cl$, 0.5% glucose, 0.5% Casamino acids, 1 mM $MgSO_4$, 0.1mM $CaCl_2$, 4 µg/ml vitamin $B_1$, pH 7.4) supplemented with 50 µg/ml ampicillin and cultivation was carried out at 30°C for 24 hours.

Cells were collected by centrifuging the culture broth at 7,000 rpm for 5 minutes and dissolved in Laemmli's sample buffer, and the solution was heated and then subjected to SDS-polyacrylamide gel electrophoresis by the method of Laemmli, followed by staining with Coomassie Brilliant Blue. As a result, a polypeptide band was detected at the site corresponding to a molecular weight of about 12,600 (cf. Fig. 7A). For the pSGHE1-free W3110 strA strain of Escherichia coli, the corresponding band could not be detected. This indicates that the pSGHE1-carrying Escherichia coli W3110 strA strain did produce sGHE1.

Example 10: Production of sGHEC-2 in Escherichia coli with the plasmid pSGHEC-2 introduced thereinto

Using pSGHEC-2 obtained in Example 4, Escherichia coli W3110 strA (FERM BP-732) was transformed by the method described in Example 3. An $Ap^r$ colony obtained was inoculated into LG medium in the same manner as in Example 9 and cultured at 30°C for 16 hours.

A 200-µl portion of this culture was inoculated into 10 ml of MCG medium supplemented with 50 µg/ml ampicillin and cultivation was carried out at 30°C for 24 hours. Cells were collected by centrifuging the culture broth at 7,000 rpm for 5 minutes and dissolved in Laemmli's sample buffer. The solution was heated and then subjected to SDS-polyacrylamide gel electrophoresis, followed by staining with Coomassie Brilliant Blue, whereupon a polypeptide band was detected at a site corresponding to a molecular weight of about 14,800 (cf. Fig. 7B). For the pSGHEC-2-free Escherichia coli W3110 strA strain, the corresponding band could not be detected. This indicates that the pSGHEC-2-carrying Escherichia coli W31l0 strA strain did produce the fused antigen polypeptide sGHEC-2 composed of the HCV-associated C-2 antigen and sGH.

Example 11: Production of a fused antigen polypeptide in Escherichia coli with the plasmid pSGHEC-14 introduced thereinto.

Using pSGHEC-14 obtained in Example 5, Escherichia coli W3110 strA (FERM BP-732) was transformed by the method described in Example 3. An $Ap^r$ colony obtained was inoculated into LG medium in the same manner as in Example 9 and cultured at 30°C for 16 hours.

A 200-µl portion of this culture was inoculated into 10 ml of MCG medium supplemented with 50 µg/ml ampicillin and cultured at 30°C for 24 hours. Cells were collected by centrifuging the culture at 7,000 rpm for 5 minutes and dissolved in Laemmli's sample buffer, and the solution was heated and then subjected to SDS-polyacrylamide gel electrophoresis, followed by staining with Coomassie Brilliant Blue, whereupon a polypeptide band was detected at a site corresponding to a molecular weight of about 17,100 (cf. Fig. 7C). For the pSGHEC-14-free Escherichia coli W3110 strA strain, the corresponding band was absent. This indicates that the pSGHEC-14-carrying Escherichia coli W3110 strA strain did produce the fused antigen polypeptide sGHEC-14 composed of the HCV-associated C-14 antigen and sGH.

Example 12: Production of a fused antigen polypeptide in Escherichia coli with the plasmid pSGHEC-18S introduced thereinto

Using pSGHEC-18S obtained in Example 7, Escherichia coli W3110 strA (FERM BP-732) was transformed by the method described in Example 3. An $Ap^r$ colony obtained was inoculated into LG medium in the same manner as in Example 9 and cultured at 30°C for 16 hours.

A 200-µl portion of this culture was inoculated into 10 ml of MCG medium [0.6% $Na_2HPO_4$, 0.3% $KH_2PO_4$, 0.5% NaCl, 0.5% Casamino acids, 1 mM $MgSO_4$, 4 µg/ml vitamin $B_1$, pH 7.4] containing 25 µg/ml tryptophan and 30 µg/ml ampicillin and cultured at 30°C for 4 to 8 hours. Then, 3β-indoleacrylic acid (hereinafter abbreviated as IAA), a tryptophan inducer, was added to a concentration of 10 µg/ml and cultivation was further continued for 2 to 12 hours.

Cells were collected by centrifuging the culture broth at 7,000 rpm for 5 minutes and dissolved in Laemmlin's sample buffer. The solution was heated and then subjected to SDS-polyacrylamide gel electrophoresis, followed by staining with Coomassie Brilliant Blue, whereupon a polypeptide band was detected at a site corresponding to a molecular weight of about 20,400 (cf. Fig. 7D). For the pSGHEC-18S-free Escherichia coli W3110 strA strain, the corresponding band was absent. This means that the pSGHEC-18S-carrying Escherichia coli W3110 strA strain did produce the fused antigen polypeptide sGHEC-18S composed of the HCV-associated C-18 antigen and sGH.

Example 13: Production of a fused antigen polypeptide in Escherichia coli with the plasmid pSGHEC-1414 introduced thereinto

Using pSGHEC-1414 obtained in Example 8, Escherichia coli W3110 strA (FERM BP-732) was transformed by the method described in Example 3. An $Ap^r$ colony obtained was inoculated into LG medium in the same manner as in Example 9 and cultured at 30°C for 16 hours. A 200-µl portion of this culture was inoculated into 10 ml of MCG medium supplemented with 50 µg/ml ampicillin and cultured at 30°C for 24 hours. Cells were collect by centrifuging the culture broth at 7,000 rpm for 5 minutes and dissolved in Laemmli's sample buffer. The solution was heated and then subjected to SDS-polyacrylamide gel electrophoresis, followed by staining with Coomassie Brilliant Blue, whereupon a polypeptide band was detected at a site corresponding to a molecular weight of about 21,700 (cf. Fig. 7E). The pSGHEC-1414-free Escherichia coli W3110 strA strain did not give the corresponding band. This means that the pSGHEC-1414-carrying Escherichia coli W3110 strA strain did produce the fused antigen polypeptide sGHEC-1414 composed of 2 molecules of the HCV-associated C-14 antigen and sGH.

Example 14: Purification of the sGHEC-14 polypeptide produced in Escherichia coli

The culture broth obtained in Example 11 was centrifuged at 8,000 rpm for 40 minutes for cell harvesting. The cells collected were washed with 30 mM Tris-HCl buffer (pH 7.5) containing 30 mM NaCl. sGHEC-14 was expressed in the form of granules intracellularly in Escherichia coli. The granular sGHEC-14 was purified from the washed cells to a purity of 90% by the method described in JP-A-60-244259. The granules (800 mg) were solubilized in 70% formic acid (final volume 40 ml) and, after 20 minutes of centrifugation at 12,000 rpm, sGHEC-14 was purified by reversed-phase HPLC. The procedual conditions of reversed-phase HPLC were as follows:

Column: Hi-PORE $C_4$, 21.5 mm x 25 cm (bio-Rad)
Solution A: 0.1% trifluoroacetic acid, 15% acetonitrile
Solution B: 0.1% trifluoroacetic acid, 90% acetonitrile
Gradient:

Table 1

| Time (min) | Solution A (%) | Solution B (%) |
|---|---|---|
| 0 | 100 | 0 |
| 60 | 0 | 100 |
| 70 | 0 | 100 |
| 80 | 100 | 0 |
| 100 | 100 | 0 |

Flow rate: 20 ml/min
Detection: Ultraviolet absorption at 220 nm
Column temperature: 35 °C

In the above reversed-phase HPLC, sGHEC-14 was eluted 26 to 28 minutes after start of gradient application. This eluate fraction (40 ml) was distributed in 1-ml portions into vials and lyophilized to give a standard sample of sGHEC-14. The contents of one vial were dissolved in 1 ml of 0.1% trifluoroacetic acid and analyzed by SDS-polyacrylamide gel electrophoresis using a protein assay kit (Bio-Rad). The protein concentration was 0.2 to 0.6 mg/ml (in the case of sGHEC-14, 0.44 mg/ml, calculated as bovine serum albumin) and the purity was not less than 90%.

sGHE1, sGHEC-2, sGHEC-18S and sGHEC-1414 can be purified in the same manner.

Example 15: Anti-HCV antibody detection using HCV-associated fused antigen polypeptides

Human sera were used as samples and Western blotting (hereinafter abbreviated as WB) was performed [Burnett: Anal. Biochem., 112, 195 (1981)].

The C-14 fused antigen polypeptide purified in Example 14 was used as the HCV-associated fused antigen.

The purified C-14 fused antigen polypeptide (100 µg) was electrophoresed on an SDS-polyacrylamide gel and the same gel was subjected to electrical blotting onto a nitrocellulose membrane [Towbin: Proc. Natl. Acad. Sci. U.S.A., 76, 4350 (1979)]. After blotting, the membrane was immersed in a blotting solution [10 mM phosphate buffer (pH 7.2), 500 mM NaCl, 5% skimmed milk, 1 drop antifoam) and shaken at room temperature for 2 hours. After shaking, the nitrocellulose membrane was cut into strips so that the protein quantity amounted to 3 µg/strip. Each strip was immersed in 3 ml of the blotting solution, 30 µl of human serum was added, and the whole was allowed to stand overnight at room temperature. Then the filter was washed with Tween-PBS [10 mM phosphate buffer (pH 7.2), 500 mM NaCl, 0.05% Tween 20], an anti-human immunoglobulin-peroxidase conjugate was added, and the reaction was conducted at room temperature for 2 hours. After the subsequent washing with Tween-PBS and final washing with PBS, a color developer system was added. The color developer system comprises a solution of 60 mg of 4-chloro-1-naphthol (Bio-Rad) in cold methanol and a mixture of 100 ml of PBS and 60 µl of aqueous hydrogen peroxide. The color development reaction was carried out for 30 minutes under protection from light and terminated by washing with water.

The sera used were as shown below. The abbreviation PIB stands for plaque immunoblotting [Arima et al.: Nippon Rinsho, 48, 27-32 (1990)] and the Chiron method is the method described by G. Kuo et al. in Science, 244, 362-364 (1989). Sera (1) and (2): patients' sera C-14 (-) and C-18 (+) in PIB; (3) through (10): patients' sera C-14 (+) in PIB; (11) through (15): patients' sera C-14 (-) in PIB; (1E) through (14E): patients' sera C-14 (-) in PIB; (15E): serum C-14 (-) in PIB, and (+) by the Chiron method; (16E): serum C-14 (-) in PIB, and (-) by the Chiron method. The results of Western blotting for sera (1) to (15) are shown in Table 2. The results of Western blotting for sera (1E) to (16E) are shown in Table 3.

With sera (1) to (10), the C-14 assay results obtained by the prior art PIB method and the results of the WB assay according to the invention showed very good correlationship. For sera (11) to (15) and (1E) to (16E), the WB assay using the fused antigen polypeptide according to the invention was successful in positively assaying the samples which showed apparent negativity in the C-14 assay by the prior art PIB method. As regards serum (16E), the WB method according to the invention could show positivity of the sample judged to be negative by the Chiron method. It was also found that when WB is carried out using the sGH polypeptide used for fusion alone, the polypeptide is not reactive with sera at all. Therefore, the fused antigen polypeptides are believed to be very effective in using serodiagnosis for detecting anti-HCV antibodies.

Table 2
Comparative assay of patients' sera

| Serum | PIB | WB | Diagnosis |
|---|---|---|---|
| 1 | - | - | Chronic hepatitis |
| 2 | - | - | |
| 3 | + | + | Chronic hepatitis |
| 4 | + | + | Chronic hepatitis |
| 5 | + | + | Chronic hepatitis |
| 6 | + | + | Chronic hepatitis |
| 7 | + | + | Chronic hepatitis |
| 8 | + | + | Hepatic cirrhosis |
| 9 | + | + | Chronic hepatitis |
| 10 | + | + | Chronic hepatitis |
| 11 | - | - | SG |
| 12 | - | + | Hepatic cirrhosis |
| 13 | - | - | HBV |
| 14 | - | - | HBV |
| 15 | - | + | Hepatic cirrhosis |

SG : Sjögren's syndrome
HBV: Hepatitis B virus carrier

Table 3

Comparative assay of patients' sera

| Serum | Diagnosis | PIB | WB | Chiron |
|---|---|---|---|---|
| 1E | Hepatic cirrhosis | - | + | |
| 2E | | - | + | |
| 3E | Convalescence phase C | - | + | |
| 4E | Hepatic cirrhosis | - | + | |
| 5E | Hepatic cirrhosis | - | + | |
| 6E | Hepatic cirrhosis | - | + | |
| 7E | Acute hepatitis | - | - | |
| 8E | Hepatic cirrhosis | - | - | |
| 9E | Chronic hepatitis | - | + | |
| 10E | Acute hepatitis | - | + | |
| 11E | Acute hepatitis | - | - | |
| 12E | Hepatic cirrhosis | - | + | |
| 13E | Hepatic cirrhosis | - | + | |
| 14E | Chronic hepatitis | - | + | |
| 15E | | - | + | + |
| 16E | | - | + | - |

Example 16: Production of DNA-29 through DNA-36

DNA-29 to DNA-36 were synthesized in the same manner as in Example 1.

Example 17: Construction of the plasmid pSGHEC-14A

The plasmid pSGHE1 (2 μg) obtained in Example 3 was dissolved in 30 μl of the restriction enzyme reaction buffer (100 mM NaCl) mentioned in Example 2, 10 units of EcoRI (Takara Shuzo) was added, and digestion was carried out at 37°C for 2 hours. To this reaction mixture was then added 1 unit of bacterial alkaline phosphatase (Takara Shuzo), and the reaction was carried out at 65°C for 30 minutes. Then, 10 units of HindIII (Takara Shuzo) and 10 units of PvuI (Takara Shuzo) were added, and further digestion was effected at 37°C for 2 hours. In parallel, pSGHE1 was digested with 10 units of HindIII (Takara Shuzo) and 10 units of PvuI (Takara Shuzo) at 37°C for 2 hours.

These reaction mixtures were subjected to electrophoresis on an agarose gel containing ethidium bromide and, using ultraviolet light (wavelength 302 nm) for detection, gel portions containing DNA fragments of about 320 bp, about 850 bp and about 1.8 kb were excised. Phenol (0.5 ml) was added to each gel section and, after freezing and thawing, the aqueous layer was separated and washed with chloroform. The above-mentioned DNA fragments were then recovered by ethanol precipitation.

DNA-29 to DNA-34 (10 picomoles each) were respectively dissolved in 30 $\mu$l of T4 polynucleotide kinase reaction buffer [50 mM Tris-HCl (pH 7.5), 10 mM $MgCl_2$, 5 mM DTT, 1 mM ATP, 0.1 mM spermidine, 0.1 mM EDTA], 3 units of T4 polynucleotide kinase (Takara Shuzo) was added to each solution, and the phosphorylation reaction was conducted at 37° C for 40 minutes. The enzyme was then inactivated by 15 minutes of heating at 65° C. The reaction mixture (4 $\mu$l each) were combined, the DNA fragments obtained as described above (0.08 picomole each) were added, the mixture was adjusted to the composition of T4 ligase reaction buffer [28 mM Tris-HCl (pH 7.5), 9 mM $MgCl_2$, 10 mM DTT, 0.03 mM EDTA, 0.7 mM ATP, 0.03 mM spermidine] and 175 units of T4 DNA ligase (Takara Shuzo) was added. The final volume was made 30 $\mu$l. The ligation reaction was carried out at 4° C for 16 hours.

Using this reaction mixture, Escherichia coli HB101 was transformed by the method of Cohen et al. to give ampicillin-resistant ($Ap^r$) colonies. Plasmid DNA recovery from one of these colonies by the alkaline treatment method gave pSGHEC-14A. The structure of pSGHEC-14A was confirmed by cleavage with BglII, PvuI, HindIII and EcoRI followed by agarose gel electrophoresis. The restriction enzyme cleavage reaction was carried out in a solution containing 10 mM Tris-HCl (pH 7.5), 7 mM $MgCl_2$ and 6 mM 2-mercaptoethanol with NaCl added to an optimum concentration (0 to 200 mM) for the enzyme used.

The pSGHEC-14A-carrying Escherichia coli strain has been deposited with the Fermentation Research Institute (FRI), Agency of Industrial Science and Technology since February 1, 1991 under the Budapest Treaty under the designation and deposit number of Escherichia coli SGHEC14A and FERM BP-3261, respectively.

Example 18 Construction of the plasmid pSGHEC-14B

The plasmid pSGHE1 (2 $\mu$g) obtained in Example 3 was dissolved in 30 $\mu$l of the restriction enzyme reaction buffer (100 mM NaCl) mentioned in Example 2, 10 units of EcoRI (Takara Shuzo) was added, and the digestion reaction was carried out at 37° C for 2 hours. Then, 1 unit of bacterial alkaline phosphatase (Takara Shuzo) was added to this reaction mixture and the reaction was conducted at 65° C for 30 minutes. Then, 10 units of HindIII (Takara Shuzo) and 10 units of PvuI (Takara Shuzo) were added, and the digestion reaction was conducted at 37° C for 2 hours. In parallel, pSGHE1 was digested with 10 units of HindIII (Takara Shuzo) and 10 units of PvuI (Takara Shuzo) at 37° C for 2 hours.

These reaction mixtures were subjected to electrophoresis on an agarose gel containing ethidium bromide and, using ultraviolet light (wavelength 302 nm) for detection, gel portions containing DNA fragments of about 320 bp, about 850 bp and about 1.8 kb, respectively. Phenol (0.5 ml) was added to each gel section and, after freezing and thawing, the aqeuous layer was taken and washed with chloroform. The DNA fragments mentioned above were then recovered by ethanol precipitation.

DNA-29 to DNA-32, DNA-35 and DNA-36 (10 picomoles each) were respectively dissolved in 30 $\mu$l of T4 polynucleotide kinase reaction buffer [50 mM Tris-HCl (pH 7.5), 10 mM $MgCl_2$, 5 mM DTT, 1 mM ATP, 0.1 mM spermidine, 0.1 mM EDTA] and 3 units of T4 polynucleotide kinase (Takara Shuzo) was added to each solution, and the phosphorylation reaction was carried out at 37° C for 40 minutes. The enzyme was then inactivated by 15 minutes of heating at 65° C. These reaction mixtures (4 $\mu$l each) were combined, the DNA fragments obtained as described above (0.08 picomole each) were added, the mixture was adjusted to the composition of T4 ligase reaction buffer [28 mM Tris-HCl (pH 7.5), 9 mM $MgCl_2$, 10 mM DTT, 0.03 mM EDTA, 0.7 mM ATP, 0.03 mM spermidine] and 175 units of T4 DNA ligase (Takara Shuzo) was added. The final volume was made 30 $\mu$l. Ligation was carried at 4° C for 16 hours.

Using this reaction mixture, Escherichia coli HB101 was transformed by the method of Cohen et al. to give ampicillin-resistant ($Ap^r$) colonies. Plasmid DNA recovery from one of these colonies by the alkaline treatment method gave pSGHEC-14B. The structure of pSGHEC-14B was confirmed by cleavage with BglIII, PvuI, HindIII and EcoRI followed by agarose gel electrophoresis. The restriction enzyme cleavage reaction was carried out in a solution containing 10 mM Tris-HCl (pH 7.5), 7 mM $MgCl_2$ and 6 mM 2-mercaptoethanol with NaCl added to an optimum concentration (0 to 200 mM) for the enzyme used.

The pSGHEC-14B-carrying Escherichia coli strain has been deposited with the Fermentation Research Institute (FRI), Agency of Industrial Science and Technology as of February 1, 1991 under the Budapest Treaty under the designation and deposit number of Escherichia coli SGHEC14B and FERM BP-3262, respectively.

Example 19: Production of a fused antigen polypeptide in Escherichia coli with the plasmid pSGHEC-14A introduced thereinto

Using pSGHEC-14A obtained in Example 17, Escherichia coli W3110 strA (FERM BP-732) was transformed by the method described in Example 3. An $Ap^r$ colony obtained was inoculated into LG medium in the same manner as in Example 9 and cultured at 30 ° C for 16 hours.

A 200-µl portion of this culture was inoculated into 10 ml of MCG medium supplemented with 50 µg/ml ampicillin and cultured at 30 ° C for 24 hours. Cells were collected by centrifuging the culture broth at 7,000 rpm for 5 minutes and dissolved in Laemmli's sample buffer. The solution was heated and then subjected to SDS-polyacrylamide gel electrophoresis, which was followed by staining with Coomassie Brilliant Blue. A polypeptide band was detected at a site corresponding to a molecular weight of about 17,100 (cf. Fig. 10F). With the pSGHEC-14A-free Escherichia coli W3110 strA strain, the corresponding band could not be detected. This indicates that the pSGHEC-14A-carrying Escherichia coli W3110 strA strain did produce the fused antigen polypeptide sGHEC-14A composed of the HCV-associated C-14A antigen and sGH.

Example 20: Production of a fused antigen polypeptide in Escherichia coli with the plasmid pSGHEC-14B introduced thereinto

Using pSGHEC-14B obtained in Example 18, Escherichia coli W3110 strA (FERM BP-732) was transformed by the method described in Example 3. An $Ap^r$ colony obtained was inoculated into LG medium in the same manner as in Example 9 and cultured at 30 ° C for 16 hours.

A 200-µl portion of this culture was inoculated into 10 ml of MCG medium supplemented with 50 µg/ml ampicillin and cultured at 30 ° C for 24 hours. Cells were collected by centrifuging the culture broth at 7,000 rpm for 5 minutes and dissolved in Laemmli's sample buffer. The solution was heated and then subjected to SDS-polyacrylamide gel electrophoresis, which was followed by staining with Coomassie Brilliant Blue. A polypeptide band was detected at a site corresponding to a molecular weight of about 16,500 (cf. Fig. 10G). The pSGHEC-14B-free Escherichia coli W3110 strA strain did not give the corresponding band. This indicates that the pSGHEC-14B-carrying Escherichia coli W31l0 strA strain did produce the fused antigen polypeptide sGHEC-14B composed of the HCV-associated C-14B antigen and sGH.

Example 21: Purification of the polypeptides sGHEC-14A and sGHEC-14B produced in Escherichia coli

sGHEC-14A and sGHEC-14B can be purified in the same manner as in Example 14.

Industrial Applicability

According to the invention, it is possible to produce fused antigen polypeptides in which an antigen polypeptide is fused with a heterologous polypeptide can be produced in large quantities. Such fused antigen polypeptides can be used for precise serodiagnosis of hepatitis C virus-infected patients.

"Sequence Listing"

SEQ ID NO: 1

SEQUENCE LENGTH: 66 base pairs

SEQUENCE TYPE: nucleic acid

STRANDEDNESS: double

TOPOLOGY: linear

MOLECULE TYPE: other nucleic acid; cDNA to RNA isolated from the serum of a subject with signs of liver dysfunction other than hepatitis B (hepatitis C virus-associated antigen gene)

FEATURES:

NAME/KEY: CDS

LOCATION: 1..66

IDENTIFICATION METHOD: E

NAME/KEY: peptide

LOCATION: 1..22

IDENTIFICATION METHOD: E

SEQUENCE DESCRIPTION:

```
GAA TCC CCA ACG CGT CGG CTT GGC CCG CGC CTT GGC CGC CGA CCC GCG    48
Glu Phe Pro Thr Arg Arg Leu Gly Pro Arg Leu Gly Arg Arg Pro Ala
 1               5                  10                  15
CTG ATG GCC GTG GAA TTC                                            66
Leu Met Ala Val Glu Phe
             20
```

SEQ ID NO: 2

SEQUENCE LENGTH: 114 base pairs

SEQUENCE TYPE: nucleic acid

STRANDEDNESS: double

TOPOLOGY: linear

MOLECULE TYPE: other nucleic acid; cDNA to RNA isolated from the serum of a subject with signs of liver dysfunction other than hepatitis B (hepatitis C virus-associated antigen gene)

FEATURES:

NAME/KEY: CDS

LOCATION: 1..114

IDENTIFICATION METHOD: E

NAME/KEY: peptide

LOCATION: 1..38

IDENTIFICATION METHOD: E

SEQUENCE DESCRIPTION:

```
GAA TTC CGA GAA CAA GAC CAG ATA AAA ACC AAA GAC AGA ACA CAA CAG    48
Glu Phe Arg Glu Gln Asp Gln Ile Lys Thr Lys Asp Arg Thr Gln Gln
  1               5                  10                  15
AGA AAG ACG AAA AGA AGC ACC AAT CGC AGG CGA AGC AAA AAC GAA AAA    96
Arg Lys Thr Lys Arg Ser Thr Asn Arg Arg Arg Ser Lys Asn Glu Lys
             20                  25                  30
AAA AAA AAA AAG GAA TCC                                           114
Lys Lys Lys Lys Glu Phe
         35
```

SEQ ID NO: 3

SEQUENCE LENGTH: 201 base pairs

SEQUENCE TYPE: nucleic acid

STRANDEDNESS: double

TOPOLOGY: linear

MOLECULE TYPE: other nucleic acid; cDNA to RNA isolated from the serum of a subject with signs of liver dysfunction other than hepatitis B (hepatitis C virus-associated antigen gene)

FEATURES:

NAME/KEY: CDS

LOCATION: 1..201

IDENTIFICATION METHOD: E

NAME/KEY: peptide

LOCATION: 1..67

IDENTIFICATION METHOD: E

SEQUENCE DESCRIPTION:

```
GAA TTC CAA GAA AAA AAG GGA GAA GCC AGC AAT GGA GAA GCC GAA AAC     48
Glu Phe Gln Glu Lys Lys Gly Glu Ala Ser Asn Gly Glu Ala Glu Asn
                    5                  10                  15
GAC ACA CAC AAG AAA CAA AGG AGG TAC AAA GAA AAA GAA AAA ACG GCA     96
Asp Thr His Lys Lys Gln Arg Arg Tyr Lys Glu Lys Glu Lys Thr Ala
                20                  25                  30
ACA AAT AAC CCA GGA AAG AAC AAA AAG CCA AGA GTG GGC AGA ATA AAA    144
Thr Asn Asn Pro Gly Lys Asn Lys Lys Pro Arg Val Gly Arg Ile Lys
            35                  40                  45
AAC TGG AAC CGG GAG GGA AGG AAG GAC GCA TAT CAG ATT AGA AAA AGG    192
Asn Trp Asn Arg Glu Gly Arg Lys Asp Ala Tyr Gln Ile Arg Lys Arg
        50                  55                  60
```

AGG GAA TTC 201

Arg Glu Phe

65

SEQ ID NO: 4

SEQUENCE LENGTH: 60 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..60

IDENTIFICATION METHOD: E

SEQUENCE DESCRIPTION:

AATTCCCAAC GCGTCGGCTT GGCCCGCGCC TTGGCCGCCG ACCCGCGCTG ATGGCCGTGG 60

SEQ ID NO: 5

SEQUENCE LENGTH: 60 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..60

IDENTIFICATION METHOD: E

SEQUENCE DESCRIPTION:

AATTCCACGG CCATCAGCGC GGGTCGGCGG CCAAGGCGCG GGCCAAGCCG ACGCGTTGGG 60

SEQ ID NO: 6

SEQUENCE LENGTH: 53 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..53

IDENTIFICATION METHOD: E

SEQUENCE DESCRIPTION:

AATTCCGAGA ACAAGACCAG ATAAAAACCA AAGACAGAAC ACAACAGAGA AAG 53

SEQ ID NO: 7

SEQUENCE LENGTH: 55 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..55

IDENTIFICATION METHOD: E

SEQUENCE DESCRIPTION:

TTTCGTCTTT CTCTGTTGTG TTCTGTCTTT GGTTTTTATC TGGTCTTGTT CTCGG 55

SEQ ID NO: 8

SEQUENCE LENGTH: 55 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..55

IDENTIFICATION METHOD: E

SEQUENCE DESCRIPTION:

ACGAAAAGAA GCACCAATCG CAGGCGAAGC AAAAACGAAA AAAAAAAAAA AAAGG 55

SEQ ID NO: 9

SEQUENCE LENGTH: 53 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..53

IDENTIFICATION METHOD: E

SEQUENCE DESCRIPTION:

AATTCCTTTT TTTTTTTTTT TTCGTTTTTG CTTCGCCTGC GATTGGTGCT TCT 53

SEQ ID NO: 10

SEQUENCE LENGTH: 50 base pair

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..50

IDENTIFICATION METHOD: E

SEQUENCE DESCRIPTION:

AATTCCAAGA AAAAAAGGGA GAAGCCAGCA ATGGAGAAGC CGAAAACGAC 50

SEQ ID NO: 11

SEQUENCE LENGTH: 52 base pair

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..52

IDENTIFICATION METHOD: E

SEQUENCE DESCRIPTION:

GTGTGTGTCG TTTTCGGCTT CTCCATTGCT GGCTTCTCCC TTTTTTTCTT GG 52

SEQ ID NO: 12

SEQUENCE LENGTH: 51 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..51

IDENTIFICATION METHOD: E

SEQUENCE DESCRIPTION:

ACACACAAGA AACAAAGGAG GTACAAAGAA AAAGAAAAAA CGGCAACAAA T 51

SEQ ID NO: 13

SEQUENCE LENGTH: 51 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..51

IDENTIFICATION METHOD: E

SEQUENCE DESCRIPTION:

TGGGTTATTT GTTGCCGTTT TTTCTTTTTC TTTGTACCTC CTTTGTTTCT T 51

SEQ ID NO: 14

SEQUENCE LENGTH: 51 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..51

IDENTIFICATION METHOD: E

SEQUENCE DESCRIPTION:

AACCCAGGAA AGAACAAAAA GCCAAGAGTG GGCAGAATAA AAAACTGGAA C 51

SEQ ID NO: 15

SEQUENCE LENGTH: 51 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..51

IDENTIFICATION METHOD: E

SEQUENCE DESCRIPTION:

CTCCCGGTTC CAGTTTTTTA TTCTGCCCAC TCTTGGCTTT TTGTTCTTTC C 51

SEQ ID NO: 16

SEQUENCE LENGTH: 43 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..43

IDENTIFICATION METHOD: E

SEQUENCE DESCRIPTION:

CGGGAGGGAA GGAAGGACGC ATATCAGATT AGAAAAAGGA GGG 43

SEQ ID NO: 17

SEQUENCE LENGTH: 41 bass pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..41

IDENTIFICATION METHOD: E

SEQUENCE DESCRIPTION:

AATTCCCTCC TTTTTCTAAT CTGATATGCG TCCTTCCTTC C 41

SEQ ID NO: 18

SEQUENCE LENGTH: 29 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..29

IDENTIFICATION METHOD: E

SEQUENCE DESCRIPTION:

GATCATGGAA TTCTAAGTAA GTAAATGCA 29

SEQ ID NO: 19

SEQUENCE LENGTH: 21 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..21

IDENTIFICATION METHOD: E

SEQUENCE DESCRIPTION:

TTTACTTACT TAGAATTCCA T 21

SEQ ID NO: 20

SEQUENCE LENGTH: 64 base pairs

SEQUENCE TYPE: nucleic acid

STRANDEDNESS: double

TOPOLOGY: linear

MOLECULE TYPE: other nucleic acid; cDNA to RAN isolated from the serum of a subject with signs of liver dysfunction other than hepatitis B (hepatitis C virus-associated antigen gene)

Synthetic DNA fragment formed from SEQ ID NO: 4 and 5.

FEATURES:

NAME/KEY: CDS

LOCATION: 1..64

IDENTIFICATION METHOD: E

SEQUENCE DESCRIPTION:

AATTCCCAACGCGTCGGCTTGGCCCGCGCCTTGGCCGCCGACCCGCGCTGATGGCCGTGG

GGGTTGCGCAGCCGAACCGGGCGCGGAACCGGCGGCTGGGCGCGACTACCGGCACCTTAA 64

EcoRI EcoRI

SEQ ID NO: 21

SEQUENCE LENGTH: 112 base pairs

SEQUENCE TYPE: nucleic acid

STRANDEDNESS: double

TOPOLOGY: linear

MOLECULE TYPE: other nucleic acid; cDNA to RNA isolated from the serum of a patient with signs of liver dysfunction other than hepatitis B.
Synthetic DNA fragment formed from SEQ ID NO: 6, 7, 8 and 9.

FEATURES:

NAME/KEY: CDS

LOCATION: 1..112

IDENTIFICATION METHOD: E

SEQUENCE DESCRIPTION:

```
AATTCCGAGAACAAGACCAGATAAAAACCAAAGACAGAACACAACAGAGAAAGACGAAA
    GGCTCTTGTTCTGGTCTATTTTTGGTTTCTGTCTTGTGTTGTCTCTTTCTGCTTT          59
  EcoRI
AGAAGCACCAATCGCAGGCGAAGCAAAAACGAAAAAAAAAAAAAAAAGG
TCTTCGTGGTTAGCGTCCGCTTCGTTTTTGCTTTTTTTTTTTTTTTTCCTTAA              112
                                                       EcoRI
```

SEQ ID NO: 22

SEQUENCE LENGTH: 119 base pairs

SEQUENCE TYPE: nucleic acid

STRANDEDNESS: double

TOPOLOGY: linear

MOLECULE TYPE: other nucleic acid; cDNA to RNA isolated from the serum of a patient with signs of liver dysfunction other than hepatitis B.

Synthetic DNA fragment formed from SEQ ID NO: 10, 11, 12, 13, 14, 15, 16 and 17.

FEATURES:

NAME/KEY: CDS

LOCATION: 1..199

IDENTIFICATION METHOD: E

SEQUENCE DESCRIPTION:

```
AATTCCAAGAAAAAAAGGGAGAAGCCAGCAATGGAGAAGCCGAAAACGACACACACAAG
    GGTTCTTTTTTTCCCTCTTCGGTCGTTACCTCTTCGGCTTTTGCTGTGTGTGTTC          59
  EcoRI
AAACAAAGGAGGTACAAAGAAAAAGAAAAAACGGCAACAAATAACCCAGGAAAGAACAAA
TTTGTTTCCTCCATGTTTCTTTTTCTTTTTTGCCGTTGTTTATTGGGTCCTTTCTTGTTT        119
AAGCCAAGAGTGGGCAGAATAAAAAACTGGAACCGGGAGGGAAGGAAGGACGCATATCAG
TTCGGTTCTCACCCGTCTTATTTTTTGACCTTGGCCCTCCCTTCCTTCCTGCGTATAGTC        179
ATTAGAAAAAGGAGGG
TAATCTTTTTCCTCCCTTAA                                                199
                  EcoRI
```

SEQ ID NO: 23

SEQUENCE LENGTH: 29 base pairs

SEQUENCE TYPE: nucleic acid

STRANDEDNESS: double

TOPOLOGY: linear

MOLECULE TYPE: other nucleic acid; linker DNA used for constructing a synthetic DNA formed from SEQ ID NO: 18 and 19.

FEATURES:

NAME/KEY: unsure

LOCATION: 1..29

IDENTIFICATION METHOD: E

SEQUENCE DESCRIPTION:

```
    MetGluPhe
GATCATGGAATTCTAAGTAAGTAAATGCA
    TACCTTAAGATTCATTCATTT                              29
  BglII  EcoRI         NsiI
```

SEQ ID NO: 24

SEQUENCE LENGTH: 337 base pairs

SEQUENCETYPE: nucleic acid

STRANDEDNESS: double

TOPOLOGY: linear

MOLECULE TYPE: other nucleic acid; obtained by joining the DNA fragment formed from SEQ ID NO: 18 and 19 to a salmon growth hormone DNA fragment.

FEATURES:

NAME/KEY: CDS

LOCATION: 1..337

IDENTIFICATION METHOD: E

NAME/KEY: piptide

LOCATION: 1..107

IDENTIFICATION METHOD: E

SEQUENCE DESCRIPTION:

```
ATG ATA GAA AAC CAA CGG CTC TTC AAC ATC GCG GTC AGT CGG GTG CAA    48
Met Ile Glu Asn Gln Arg Leu Phe Asn Ile Ala Val Ser Arg Val Gln
                    5                  10                  15
CAT CTC CAC CTA TTG GCT CAG AAA ATG TTC AAT GAC TTT GAC GGT ACC    96
His Leu His Leu Leu Ala Gln Lys Met Phe Asn Asp Phe Asp Gly Thr
               20                  25                  30
CTG TTG CCT GAT GAA CGC AGA CAG CTG AAC AAG ATA TTC CTG CTG GAC   144
Leu Leu Pro Asp Glu Arg Arg Gln Leu Asn Lys Ile Phe Leu Leu Asp
           35                  40                  45
TTC TGT AAC TCT GAC TCC ATC GTG AGC CCA GTC GAC AAG CAC GAG ACT   192
Phe Cys Asn Ser Asp Ser Ile Val Ser Pro Val Asp Lys His Glu Thr
       50                  55                  60
CAG AAG AGT TCA GTC CTG AAG CTG CTC CAC ATT TCT TTC CGT CTG ATT   240
Gln Lys Ser Ser Val Leu Lys Leu Leu His Ile Ser Phe Arg Leu Ile
 65                  70                  75                  80
GAA TCC TGG GAG TAC CCT AGC CAG ACC CTG ATC ATC TCC AAC AGC CTA   288
Glu Ser Trp Glu Tyr Pro Ser Gln Thr Leu Ile Ile Ser Asn Ser Leu
                    85                  90                  95
ATG GTC AGA AAC GCC AAC CAG ATC ATG GAA TTC TAAGTAAGTA AATGCA     337
Met Val Arg Asn Ala Asn Gln Ile Met Glu Phe _________________
              100                 105   EcoRI
```

SEQ ID NO: 25

SEQUENCE LENGTH: 381 base pairs

SEQUENCE TYPE: nucleic acid

STRANDEDNESS: double

TOPOLOGY: linear

MOLECULE TYPE: other nucleic acid; obtained by insertion of the DNA fragment formed from SEQ ID NO: 4 and 5 into the DNA fragment of SEQ ID NO: 24.

FEATURES:

NAME/KEY: CDS

LOCATION: 1..381

IDENTIFICATION METHOD: E

NAME/KEY: peptide

LOCATION: 1..127

IDENTIFICATION METHOD: E

SEQUENCE DESCRIPTION:

```
ATG ATA GAA AAC CAA CGG CTC TTC AAC ATC GCG GTC AGT CGG GTG CAA    48
Met Ile Glu Asn Gln Arg Leu Phe Asn Ile Ala Val Ser Arg Val Gln
                    5                  10                  15
CAT CTC CAC CTA TTG GCT CAG AAA ATG TTC AAT GAC TTT GAC GGT ACC    96
His Leu His Leu Leu Ala Gln Lys Met Phe Asn Asp Phe Asp Gly Thr
                20                  25                  30
CTG TTG CCT GAT GAA CGC AGA CAG CTG AAC AAG ATA TTC CTG CTG GAC   144
Leu Leu Pro Asp Glu Arg Arg Gln Leu Asn Lys Ile Phe Leu Leu Asp
            35                  40                  45
TTC TGT AAC TCT GAC TCC ATC GTG AGC CCA GTC GAC AAG CAC GAG ACT   192
Phe Cys Asn Ser Asp Ser Ile Val Ser Pro Val Asp Lys His Glu Thr
        50                  55                  60
```

```
CAG AAG AGT TCA GTC CTG AAG CTG CTC CAC ATT TCT TTC CGT CTG ATT    240
Gln Lys Ser Ser Val Leu Lys Leu Leu His Ile Ser Phe Arg Leu Ile
 65                 70                  75                  80
GAA TCC TGG GAG TAC CCT AGC CAG ACC CTG ATC ATC TCC AAC AGC CTA    288
Glu Ser Trp Glu Tyr Pro Ser Gln Thr Leu Ile Ile Ser Asn Ser Leu
                85                  90                  95
ATG GTC AGA AAC GCC AAC CAG ATC ATG GAA TTC CCA ACG CGT CGG CTT    336
Met Val Arg Asn Ala Asn Gln Ile Met Glu Phe Pro Thr Arg Arg Leu
            100                 105                 110
GGC CCG CGC CTT GGC CGC CGA CCC GCG CTG ATG GCC GTG GAA TTC        381
Gly Pro Arg Leu Gly Arg Arg Pro Ala Leu Met Ala Val Glu Phe
        115                 120                 125
```

SEQ ID NO: 26

SEQUENCE LENGTH: 429 base pairs

SEQUENCE TYPE: nucleic acid

STRANDEDNESS: double

TOPOLOGY: linear

MOLECULE TYPE: other nucleic acid; obtained by insertion of the DNA fragment formed from SEQ ID NO: 6, 7, 8 and 9 into the DNA fragment of SEQ ID NO: 24.

FEATURES:

NAME/KEY: CDS

LOCATION: 1..429

IDENTIFICATION METHOD: E

NAME/KEY: peptide

LOCATION: 1..143

IDENTIFICATION METHOD: E

SEQUENCE DESCRIPTION:

```
ATG ATA GAA AAC CAA CGG CTC TTC AAC ATC GCG GTC AGT CGG GTG CAA    48
Met Ile Glu Asn Gln Arg Leu Phe Asn Ile Ala Val Ser Arg Val Gln
                  5                  10                  15
CAT CTC CAC CTA TTG GCT CAG AAA ATG TTC AAT GAC TTT GAC GGT ACC    96
His Leu His Leu Leu Ala Gln Lys Met Phe Asn Asp Phe Asp Gly Thr
             20                  25                  30
CTG TTG CCT GAT GAA CGC AGA CAG CTG AAC AAG ATA TTC CTG CTG GAC   144
Leu Leu Pro Asp Glu Arg Arg Gln Leu Asn Lys Ile Phe Leu Leu Asp
         35                  40                  45
TTC TGT AAC TCT GAC TCC ATC GTG AGC CCA GTC GAC AAG CAC GAG ACT   192
Phe Cys Asn Ser Asp Ser Ile Val Ser Pro Val Asp Lys His Glu Thr
     50                  55                  60
CAG AAG AGT TCA GTC CTG AAG CTG CTC CAC ATT TCT TTC CGT CTG ATT   240
Gln Lys Ser Ser Val Leu Lys Leu Leu His Ile Ser Phe Arg Leu Ile
 65                  70                  75                  80
GAA TCC TGG GAG TAC CCT AGC CAG ACC CTG ATC ATC TCC AAC AGC CTA   288
Glu Ser Trp Glu Tyr Pro Ser Gln Thr Leu Ile Ile Ser Asn Ser Leu
                 85                  90                  95
ATG GTC AGA AAC GCC AAC CAG ATC ATG GAA TTC CGA GAA CAA GAC CAG   338
Met Val Arg Asn Ala Asn Gln Ile Met Glu Phe Arg Glu Gln Asp Gln
            100                 105                 110
```

ATA AAA ACC AAA GAC AGA ACA CAA CAG AGA AAG ACG AAA AGA AGC ACC 384
Ile Lys Thr Lys Asp Arg Thr Gln Gln Arg Lys Thr Lys Arg Ser Thr
115 120 125

AAT CGC AGG CGA AGC AAA AAC GAA AAA AAA AAA AAA AAG GAA TTC 429
Asn Arg Arg Arg Ser Lys Asn Glu Lys Lys Lys Lys Lys Glu Phe
130 135 140

SEQ ID NO: 27

SEQUENCE LENGTH: 516 base pairs

SEQUENCE TYPE: nucleic acid

STRANDEDNESS: double

TOPOLOGY: linear

MOLECULE TYPE: other nucleic acid; obtained by insertion of the DNA fragment formed from SEQ ID NO: 10, 11, 12, 13, 14, 15, 16 and 17 into the DNA fragment of SEQ ID NO: 24.

FEATURES:

NAME/KEY: CDS
LOCATION: 1..516
IDENTIFICATION METHOD: E
NAME/KEY: peptide
LOCATION: 1..172
IDENTIFICATION METHOD: E

SEQUENCE DESCRIPTION:

ATG ATA GAA AAC CAA CGG CTC TTC AAC ATC GCG GTC AGT CGG GTG CAA 48
Met Ile Glu Asn Gln Arg Leu Phe Asn Ile Ala Val Ser Arg Val Gln
5 10 15

```
CAT CTC CAC CTA TTG GCT CAG AAA ATG TTC AAT GAC TTT GAC GGT ACC    96
His Leu His Leu Leu Ala Gln Lys Met Phe Asn Asp Phe Asp Gly Thr
             20                  25                  30
CTG TTG CCT GAT GAA CGC AGA CAG CTG AAC AAG ATA TTC CTG CTG GAC   144
Leu Leu Pro Asp Glu Arg Arg Gln Leu Asn Lys Ile Phe Leu Leu Asp
         35                  40                  45
TTC TGT AAC TCT GAC TCC ATC GTG AGC CCA GTC GAC AAG CAC GAG ACT   192
Phe Cys Asn Ser Asp Ser Ile Val Ser Pro Val Asp Lys His Glu Thr
     50                  55                  60
CAG AAG AGT TCA GTC CTG AAG CTG CTC CAC ATT TCT TTC CGT CTG ATT   240
Gln Lys Ser Ser Val Leu Lys Leu Leu His Ile Ser Phe Arg Leu Ile
 65                  70                  75                  80
GAA TCC TGG GAG TAC CCT AGC CAG ACC CTG ATC ATC TCC AAC AGC CTA   288
Glu Ser Trp Glu Tyr Pro Ser Gln Thr Leu Ile Ile Ser Asn Ser Leu
                 85                  90                  95
ATG GTC AGA AAC GCC AAC CAG ATC ATG GAA TTC CAA GAA AAA AAG GGA   336
Met Val Arg Asn Ala Asn Gln Ile Met Glu Phe Gln Glu Lys Lys Gly
            100                 105                 110
GAA GCC AGC AAT GGA GAA GCC GAA AAC GAC ACA CAC AAG AAA CAA AGG   384
Glu Ala Ser Asn Gly Glu Ala Glu Asn Asp Thr His Lys Lys Gln Arg
        115                 120                 125
```

```
AGG TAC AAA GAA AAA GAA AAA ACG GCA ACA AAT AAC CCA GGA AAG AAC   432
Arg Tyr Lys Glu Lys Glu Lys Thr Ala Thr Asn Asn Pro Gly Lys Asn
    130                 135                 140
AAA AAG CCA AGA GTG GGC AGA ATA AAA AAC TGG AAC CGG GAG GGA AGG   480
Lys Lys Pro Arg Val Gly Arg Ile Lys Asn Trp Asn Arg Glu Gly Arg
145                 150                 155                 160
AAG GAC GCA TAT CAG ATT AGA AAA AGG AGG GAA TTC                   516
Lys Asp Ala Tyr Gln Ile Arg Lys Arg Arg Glu Phe
                165                 170
```

SEQ ID NO: 28

SEQUENCE LENGTH: 537 base pairs

SEQUENCE TYPE: nucleic acid

STRANDEDNESS: double

TOPOLOGY: linear

MOLECULE TYPE: other nucleic acid; obtained by insertion of the DNA fragment (tandem) formed from SEQ ID NO: 6, 7, 8 and 9 into the DNA fragment of SEQ ID NO: 24.

FEATURES:

NAME/KEY: CDS

LOCATION: 1..537

IDENTIFICATION METHOD: E

NAME/KEY: CDS

LOCATION: 1..537

IDENTIFICATION METHOD: E

SEQUENCE DESCRIPTION:

ATG ATA GAA AAC CAA CGG CTC TTC AAC ATC GCG GTC AGT CGG GTG CAA 48
Met Ile Glu Asn Gln Arg Leu Phe Asn Ile Ala Val Ser Arg Val Gln
5 10 15

CAT CTC CAC CTA TTG GCT CAG AAA ATG TTC AAT GAC TTT GAC GGT ACC 96
His Leu His Leu Leu Ala Gln Lys Met Phe Asn Asp Phe Asp Gly Thr
20 25 30

CTG TTG CCT GAT GAA CGC AGA CAG CTG AAC AAG ATA TTC CTG CTG GAC 144
Leu Leu Pro Asp Glu Arg Arg Gln Leu Asn Lys Ile Phe Leu Leu Asp
35 40 45

TTC TGT AAC TCT GAC TCC ATC GTG AGC CCA GTC GAC AAG CAC GAG ACT 192
Phe Cys Asn Ser Asp Ser Ile Val Ser Pro Val Asp Lys His Glu Thr
50 55 60

CAG AAG AGT TCA GTC CTG AAG CTG CTC CAC ATT TCT TTC CGT CTG ATT 240
Gln Lys Ser Ser Val Leu Lys Leu Leu His Ile Ser Phe Arg Leu Ile
65 70 75 80

GAA TCC TGG GAG TAC CCT AGC CAG ACC CTG ATC ATC TCC AAC AGC CTA 288
Glu Ser Trp Glu Tyr Pro Ser Gln Thr Leu Ile Ile Ser Asn Ser Leu
85 90 95

ATG GTC AGA AAC GCC AAC CAG ATC ATG GAA TTC CGA GAA CAA GAC CAG 338
Met Val Arg Asn Ala Asn Gln Ile Met Glu Phe Arg Glu Gln Asp Gln
100 105 110

ATA AAA ACC AAA GAC AGA ACA CAA CAG AGA AAG ACG AAA AGA AGC ACC 384
Ile Lys Thr Lys Asp Arg Thr Gln Gln Arg Lys Thr Lys Arg Ser Thr
115 120 125

```
AAT CGC AGG CGA AGC AAA AAC GAA AAA AAA AAA AAA AAG GAA TTC CGA    432
Asn Arg Arg Arg Ser Lys Asn Glu Lys Lys Lys Lys Lys Glu Phe Arg
    130                 135                 140
GAA CAA GAC CAG ATA AAA ACC AAA GAC AGA ACA CAA CAG AGA AAG ACG    480
Glu Gln Asp Gln Ile Lys Thr Lys Asp Arg Thr Gln Gln Arg Lys Thr
145                 150                 155                 160
AAA AGA AGC ACC AAT CGC AGG CGA AGC AAA AAC GAA AAA AAA AAA AAA    528
Lys Arg Ser Thr Asn Arg Arg Arg Ser Lys Asn Glu Lys Lys Lys Lys
                165                 170                 175
AAG GAA TTC                                                        537
Lys Glu Phe
```

SEQ ID NO: 29

SEQUENCE LENGTH: 40 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..40

IDENTIFICATION METHOD: E

SEQUENCE DESCRIPTION:

AATTCCGAGA ACAGGACCAG ATAAAAACCA AGGACAGAAC 40

SEQ ID NO: 30

SEQUENCE LENGTH: 43 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..43

IDENTIFICATION METHOD: E

SEQUENCE DESCRIPTION:

CTGTTGTGTT CTGTCCTTGG TTTTTATCTG GTCCTGTTCT CGG 43

SEQ ID NO: 31

SEQUENCE LENGTH: 38 base pairs

TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..38

IDENTIFICATION METHOD: E

SEQUENCE DESCRIPTION:

ACAACAGAGA AAGACGAAAA GAAGTACTAA TCGCAGGC 38

SEQ ID NO: 32

SEQUENCE LENGTH: 36 base pairs

TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..36

IDENTIFICATION METHOD: E

SEQUENCE DESCRIPTION:

GCTTCGCCTG CGATTAGTAC TTCTTTTCGT CTTTCT 36

SEQ ID NO: 33

SEQUENCE LENGTH: 30 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..30

IDENTIFICATION METHOD: E

SEQUENCE DESCRIPTION:

GAAGCAAAAA CGAAAAAAAG AAAAAAAAGG 30

SEQ ID NO: 34

SEQUENCE LENGTH: 29 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..29

IDENTIFICATION METHOD: E

SEQUENCE DESCRIPTION:

AATTCCTTTT TTTTCTTTTT TTCGTTTTT 29

SEQ ID NO: 35

SEQUENCE LENGTH: 15 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..15

IDENTIFICATION METHOD: E

SEQUENCE DESCRIPTION:

GAAGCAAAAA CGAAG 15

SEQ ID NO: 36

SEQUENCE LENGTH: 14 base pairs

SEQUENCE TYPE: nucleic acid

STRANDEDNESS: single

TOPOLOGY: linear

MOLECULE TYPE: other nucleic acid; synthetic DNA

FEATURES:

NAME/KEY: unsure

LOCATION: 1..14

IDENTIFICATION METHOD: E

SEQUENCE DESCRIPTION:

AATTCTTCGT TTTT 14

SEQ ID NO: 37

SEQUENCE LENGTH: 112 base pairs

SEQUENCE TYPE: nucleic acid

STRANDEDNESS: double

TOPOLOGY: linear

MOLECULE TYPE: other nucleic acid; DNA fragment formed from SEQ ID NO: 29, 30, 31, 32, 33 and 34.

FEATURES:

NAME/KEY: CDS

LOCATION: 1..112

IDENTIFICATION METHOD: E

SEQUENCE DESCRIPTION:

AATTCCGAGAACAGGACCAGATAAAAACCAAGGACAGAACACAACAGAGAAAGACGAAA
GGCTCTTGTCCTGGTCTATTTTTGGTTCCTGTCTTGTGTTGTCTCTTTCTGCTTT 59
EcoRI
AGAAGTACTAATCGCAGGCGAAGCAAAAACGAAAAAAAGAAAAAAAAGG
TCTTCATGATTAGCGTCCGCTTCGTTTTTGCTTTTTTTCTTTTTTTTCCTTAA 112
EcoRI

SEQ ID NO: 38

SEQUENCE LENGTH: 97 base pairs

SEQUENCE TYPE: nucleic acid

STRANDEDNESS: double

TOPOLOGY: linear

MOLECULE TYPE: other nucleic acid; DNA fragment formed from SEQ ID NO: 29, 30 31, 32, 35 and 36.

FEATURES:

NAME/KEY: CDS

LOCATION: 1..97

IDENTIFICATION METHOD: E

SEQUENCE DESCRIPTION:

AATTCCGAGAACAGGACCAGATAAAAACCAAGGACAGAACACAACAGAGAAAGACGAAA
GGCTCTTGTCCTGGTCTATTTTTGGTTCCTGTCTTGTGTTGTCTCTTTCTGCTTT 59
EcoRI
AGAAGTACTAATCGCAGGCGAAGCAAAAACGAAG
TCTTCATGATTAGCGTCCGCTTCGTTTTTGCTTCTTAA 97
EcoRI

SEQ ID NO: 39

SEQUENCE LENGTH: 429 base pairs

SEQUENCE TYPE: nucleic acid

STRANDEDNESS: double

TOPOLOGY: linear

MOLECULE TYPE: other nucleic acid; synthetic DNA fragment formed from SEQ ID NO: 29, 30, 31, 32, 33, 34.

FEATURES:

NAME/KEY: CDS

LOCATION: 1..429

IDENTIFICATION METHOD: E

NAME/KEY: peptide

LOCATION: 1..143

IDENTIFICATION METHOD: E

SEQUENCE DESCRIPTION:

```
ATG ATA GAA AAC CAA CGG CTC TTC AAC ATC GCG GTC AGT CGG GTG CAA    48
Met Ile Glu Asn Gln Arg Leu Phe Asn Ile Ala Val Ser Arg Val Gln
                    5                  10                  15
CAT CTC CAC CTA TTG GCT CAG AAA ATG TTC AAT GAC TTT GAC GGT ACC    96
His Leu His Leu Leu Ala Gln Lys Met Phe Asn Asp Phe Asp Gly Thr
               20                  25                  30
CTG TTG CCT GAT GAA CGC AGA CAG CTG AAC AAG ATA TTC CTG CTG GAC   144
Leu Leu Pro Asp Glu Arg Arg Gln Leu Asn Lys Ile Phe Leu Leu Asp
          35                  40                  45
```

TTC TGT AAC TCT GAC TCC ATC GTG AGC CCA GTC GAC AAG CAC GAG ACT 192
Phe Cys Asn Ser Asp Ser Ile Val Ser Pro Val Asp Lys His Glu Thr
50 55 60

CAG AAG AGT TCA GTC CTG AAG CTG CTC CAC ATT TCT TTC CGT CTG ATT 240
Gln Lys Ser Ser Val Leu Lys Leu Leu His Ile Ser Phe Arg Leu Ile
65 70 75 80

GAA TCC TGG GAG TAC CCT AGC CAG ACC CTG ATC ATC TCC AAC AGC CTA 288
Glu Ser Trp Glu Tyr Pro Ser Gln Thr Leu Ile Ile Ser Asn Ser Leu
85 90 95

ATG GTC AGA AAC GCC AAC CAG ATC ATG GAA TTC CGA GAA CAG GAC CAG 336
Met Val Arg Asn Ala Asn Gln Ile Met Glu Phe Arg Glu Gln Asp Gln
100 105 110

ATA AAA ACC AAG GAC AGA ACA CAA CAG AGA AAG ACG AAA AGA AGT ACT 384
Ile Lys Thr Lys Asp Arg Thr Gln Gln Arg Lys Thr Lys Arg Ser Thr
115 120 125

AAT CGC AGG CGA AGC AAA AAC GAA AAA AAG AAA AAA AAG GAA TTC 429
Asn Arg Arg Arg Ser Lys Asn Glu Lys Lys Lys Lys Lys Glu Phe
130 135 140

SEQ ID NO: 40

SEQUENCE LENGTH: 414 base pairs

SEQUENCE TYPE: nucleic acid

STRANDEDNESS: double

TOPOLOGY: linear

MOLECULE TYPE: other nucleic acid; obtained by insertion of the DNA fragment formed from SEQ ID NO: 29, 30, 31, 32, 35 and

36 into the DNA fragment of Sequence Identification No. 24.

FEATURES:

NAME/KEY: CDS

LOCATION: 1..414

IDENTIFICATION METHOD: E

NAME/KEY: peptide

LOCATION: 1..138

IDENTIFICATION METHOD: E

SEQUENCE DESCRIPTION:

```
ATG ATA GAA AAC CAA CGG CTC TTC AAC ATC GCG GTC AGT CGG GTG CAA    48
Met Ile Glu Asn Gln Arg Leu Phe Asn Ile Ala Val Ser Arg Val Gln
                    5                  10                  15
CAT CTC CAC CTA TTG GCT CAG AAA ATG TTC AAT GAC TTT GAC GGT ACC    96
His Leu His Leu Leu Ala Gln Lys Met Phe Asn Asp Phe Asp Gly Thr
                20                  25                  30
CTG TTG CCT GAT GAA CGC AGA CAG CTG AAC AAG ATA TTC CTG CTG GAC   144
Leu Leu Pro Asp Glu Arg Arg Gln Leu Asn Lys Ile Phe Leu Leu Asp
            35                  40                  45
TTC TGT AAC TCT GAC TCC ATC GTG AGC CCA GTC GAC AAG CAC GAG ACT   192
Phe Cys Asn Ser Asp Ser Ile Val Ser Pro Val Asp Lys His Glu Thr
        50                  55                  60
CAG AAG AGT TCA GTC CTG AAG CTG CTC CAC ATT TCT TTC CGT CTG ATT   240
Gln Lys Ser Ser Val Leu Lys Leu Leu His Ile Ser Phe Arg Leu Ile
 65                  70                  75                  80
```

```
GAA TCC TGG GAG TAC CCT AGC CAG ACC CTG ATC ATC TCC AAC AGC CTA   288
Glu Ser Trp Glu Tyr Pro Ser Gln Thr Leu Ile Ile Ser Asn Ser Leu
                 85                  90                  95
ATG GTC AGA AAC GCC AAC CAG ATC ATG GAA TTC CGA GAA CAG GAC CAG   336
Met Val Arg Asn Ala Asn Gln Ile Met Glu Phe Arg Glu Gln Asp Gln
            100                 105                 110
ATA AAA ACC AAG GAC AGA ACA CAA CAG AGA AAG ACG AAA AGA AGT ACT   384
Ile Lys Thr Lys Asp Arg Thr Gln Gln Arg Lys Thr Lys Arg Ser Thr
        115                 120                 125
AAT CGC AGG CGA AGC AAA AAC GAA GAA TTC                           414
Asn Arg Arg Arg Ser Lys Asn Glu Glu Phe
    130                 135
```

SEQ ID NO: 41

SEQUENCE LENGTH: 114 base pairs

SEQUENCE TYPE: nucleic acid

STRANDEDNESS: double

TOPOLOGY: linear

MOLECULE TYPE: other nucleic acid; derived from the DNA base sequence of Sequence Identification No. 2 by partial base substitution.

FEATURES:

NAME/KEY: CDS

LOCATION: 1..414

IDENTIFICATION METHOD: E

NAME/KEY: peptide

LOCATION: 1..38

IDENTIFICATION METHOD: E

SEQUENCE DESCRIPTION:

```
GAA TTC CGA GAA CAG GAC CAG ATA AAA ACC AAG GAC AGA ACA CAA CAG     48
Glu Phe Arg Glu Gln Asp Gln Ile Lys Thr Lys Asp Arg Thr Gln Gln
                    5                  10                  15
AGA AAG ACG AAA AGA AGT ACT AAT CGC AGG CGA AGC AAA AAC GAA AAA     96
Arg Lys Thr Lys Arg Ser Thr Asn Arg Arg Arg Ser Lys Asn Glu Lys
                20                  25                  30
AAG AAA AAA AAG GAA TTC                                            114
Lys Lys Lys Lys Glu Phe
            35
```

SEQ ID NO: 42

SEQUENCE LENGTH: 99 base pairs

SEQUENCE TYPE: nucleic acid

STRANDEDNESS: double

TOPOLOGY: linear

MOLECULE TYPE: other nucleic acid; derived from the DNA base sequence of Sequence Identification No. 2 by partial base substitution and partial deletion.

FEATURES:

NAME/KEY: CDS

LOCATION: 1..99

IDENTIFICATION METHOD: E

NAME/KEY: peptide

LOCATION: 1..33

IDENTIFICATION METHOD: E

SEQUENCE DESCRIPTION:

```
GAA TTC CGA GAA CAG GAC CAG ATA AAA ACC AAG GAC AGA ACA CAA CAG     48
Glu Phe Arg Glu Gln Asp Gln Ile Lys Thr Lys Asp Arg Thr Gln Gln
                5                   10                  15
AGA AAG ACG AAA AGA AGT ACT AAT CGC AGG CGA AGC AAA AAC GAA GAA     96
Arg Lys Thr Lys Arg Ser Thr Asn Arg Arg Arg Ser Lys Asn Glu Glu
            20                  25                  30
TTC                                                                 99
Phe
```

## Claims

1. A fused antigen polypeptide in which at least one hepatitis C-associated antigen-derived antigen polypeptide (hereinafter referred to briefly as hepatitis C-associated antigen polypeptide) is fused to at least one polypeptide heterologous to said antigen polypeptide (hereinafter briefly referred to as heterologous polypeptide).

2. A fused antigen polypeptide as claimed in Claim 1, wherein said heterologous polypeptide is chum salmon growth hormone polypeptide or a portion thereof, which does not specifically react with any of human serum antibodies.

3. A fused antigen polypeptide as claimed in Claim 1, wherein the hepatitis C-associated antigen polypeptide is a polypeptide having the sequence specified by SEQ ID NO: 1, 2, 3, 41 or 42.

4. A fused antigen polypeptide as claimed in Claim 1, wherein said hepatitis C-associated antigen polypeptide is bound to chum salmon growth hormone polypeptide via a methionine residue.

5. A recombinant plasmid which contains, as inserts, at least one DNA coding for a hepatitis C-associated antigen polypeptide and at least one DNA coding for a heterologous polypeptide.

6. A recombinant plasmid as claimed in Claim 5, wherein said heterologous polypeptide is chum salmon growth hormone polypeptide or a portion thereof, which does not specifically react with any of human serum antibodies.

7. A recombinant plasmid as claimed in Claim 5, wherein the hepatitis C-associated antigen polypeptide-encoding DNA is a DNA having the sequence specified under SEQ ID NO: 1, 2, 3, 41 or 42.

8. A recombinant plasmid which contains, as an insert, a DNA coding for a polypeptide composed of a hepatitis C-associated antigen polypeptide and chum salmon growth hormone polypeptide fused thereto via a methionine residue.

9. A recombinant plasmid as claimed in Claim 5, which is selected from among the plasmids pSGHEC-2, pSGHEC-14, pSGHEC-18, pSGHEC-18S, pSGHEC-1414, pSGHEC-14A and pSGHEC-14B.

10. A method of producing fused antigen polypeptides which comprises cultivating a microorganism carrying the recombinant plasmid described in any of Claims 5 to 9 in a medium to thereby cause formation and accumulation of a fused antigen polypeptide in the culture and recovering said fused antigen polypeptide from said culture.

**11.** A method as claimed in Claim 10, wherein said microorganism belongs to the species Escherichia coli.

**12.** A method of detecting an anti-hepatitis C virus (anti-HCV) antibody in a sample which comprises detecting said anti-HCV antibody by an immunological technique using, as an antigenic substance, a fused antigen polypeptide composed of a hepatitis C-associated antigen polypeptide and chum salmon growth hormone polypeptide fused thereto via a methionine residue.

**13.** A detecting method as claimed in Claim 12, wherein the immunological technique is selected from among radioimmunoassay, enzyme immunoassay, fluorescence immunoassay, reverse passive agglutination, phytohematoagglutination, particle agglutination, laser nephrometry, Western blotting and immune hemolysis (using erythrocytes or liposomes).

**14.** A detecting method as claimed in Claim 13, wherein a solid phase coated with the antigenic substance is used.

**15.** A detecting method as claimed in Claim 14, wherein said solid phase is a synthetic resin, a glass tube, a glass bead or a glass-made microtiter plate.

Fig. 1

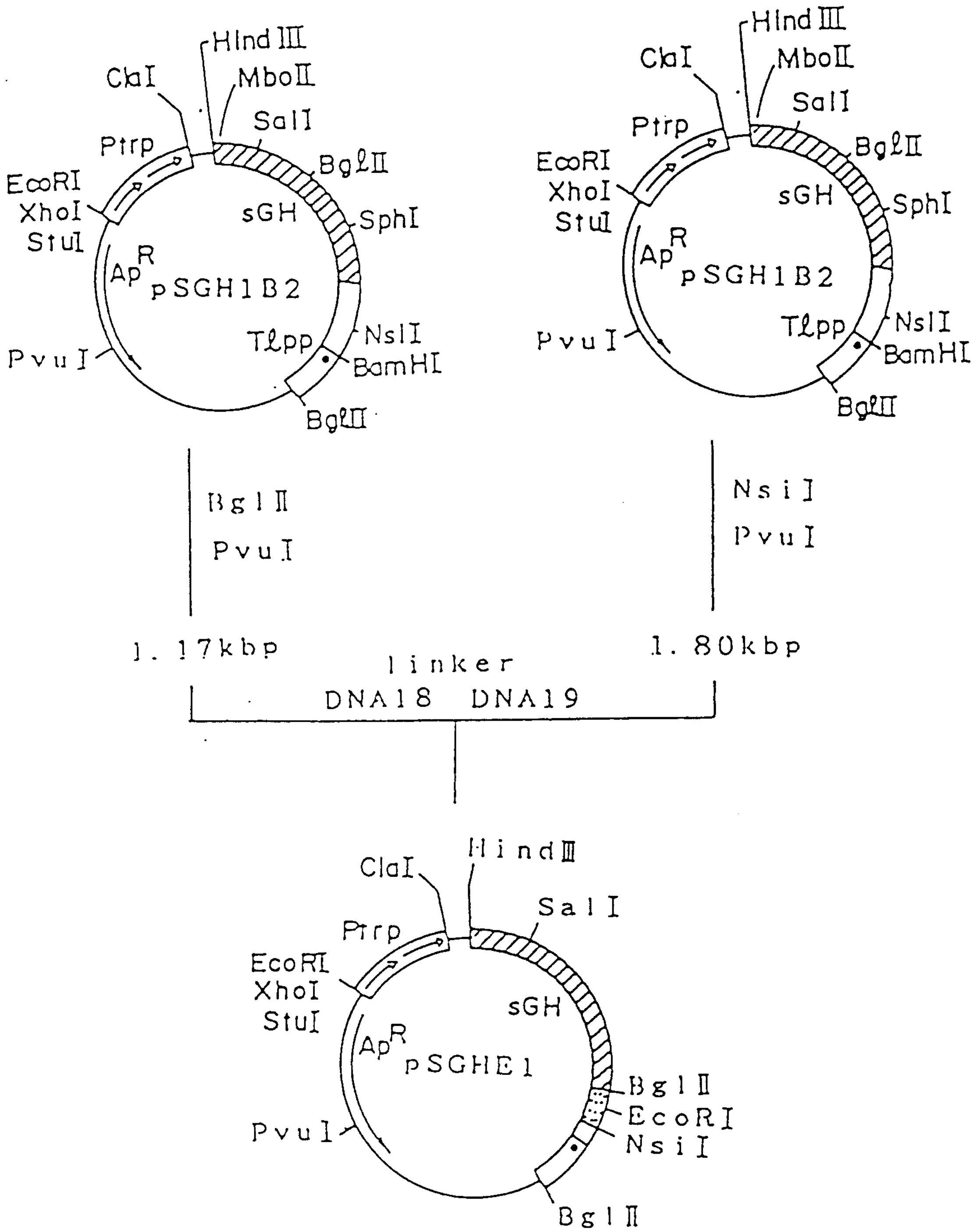
HindIII
MboII
ClaI
SalI
Ptrp
BglII
EcoRI
XhoI
StuI
sGH
SphI
ApR
pSGH1B2
TLpp
NsiI
PvuI
BamHI
BglII
BglII
PvuI
NsiI
PvuI
1.17kbp
linker
1.80kbp
DNA18 DNA19
ClaI
HindIII
SalI
Ptrp
EcoRI
XhoI
StuI
sGH
ApR
pSGHE1
BglII
EcoRI
NsiI
PvuI
BglII

Fig. 2

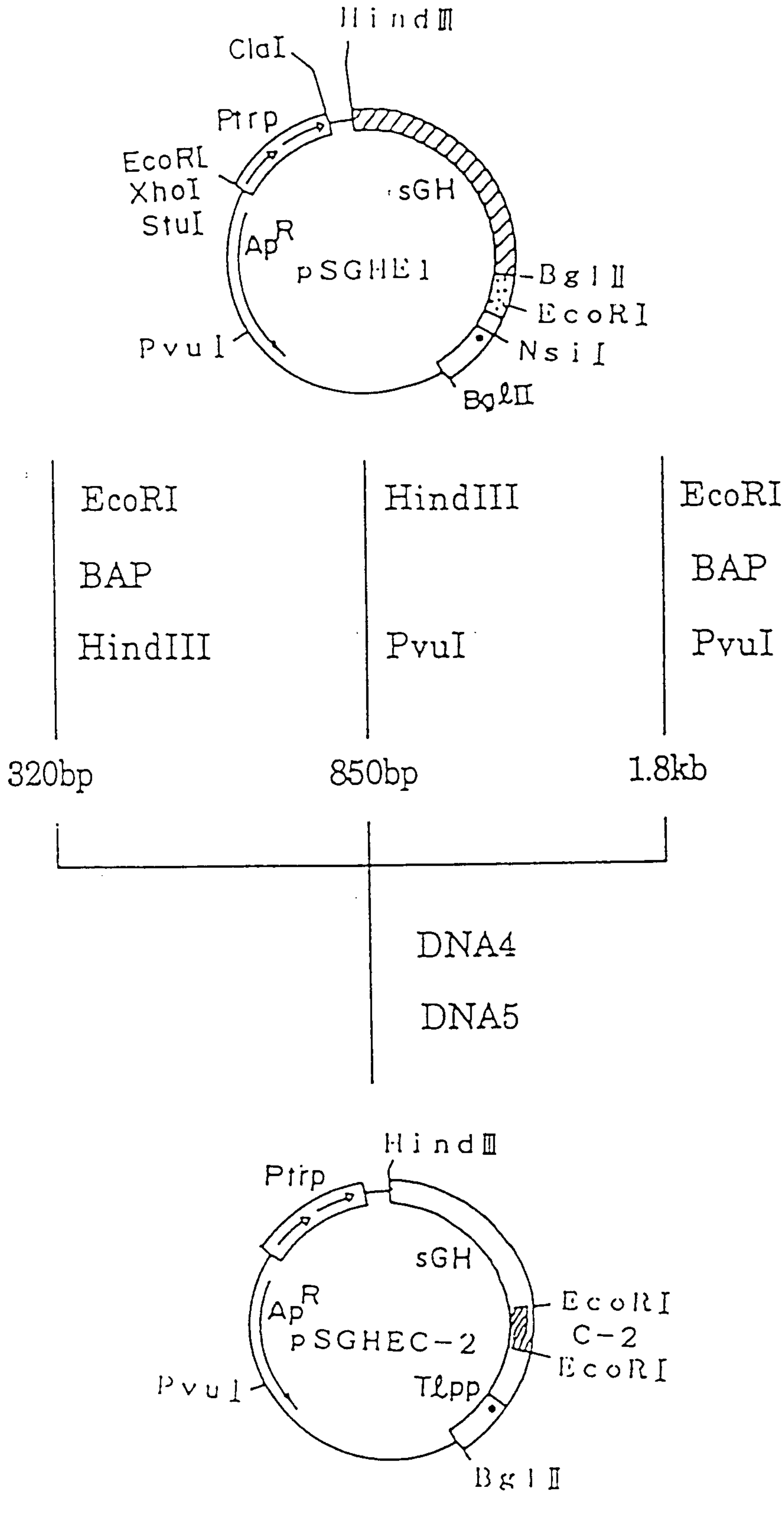
HindIII
ClaI
Ptrp
EcoRI
XhoI
StuI
sGH
ApR
pSGHE1
BglII
EcoRI
NsiI
PvuI
BglII
EcoRI
BAP
HindIII
HindIII
PvuI
EcoRI
BAP
PvuI
320bp
850bp
1.8kb
DNA4
DNA5
HindIII
Ptrp
sGH
ApR
pSGHEC-2
EcoRI
C-2
EcoRI
Tℓpp
PvuI
BglII

Fig. 3

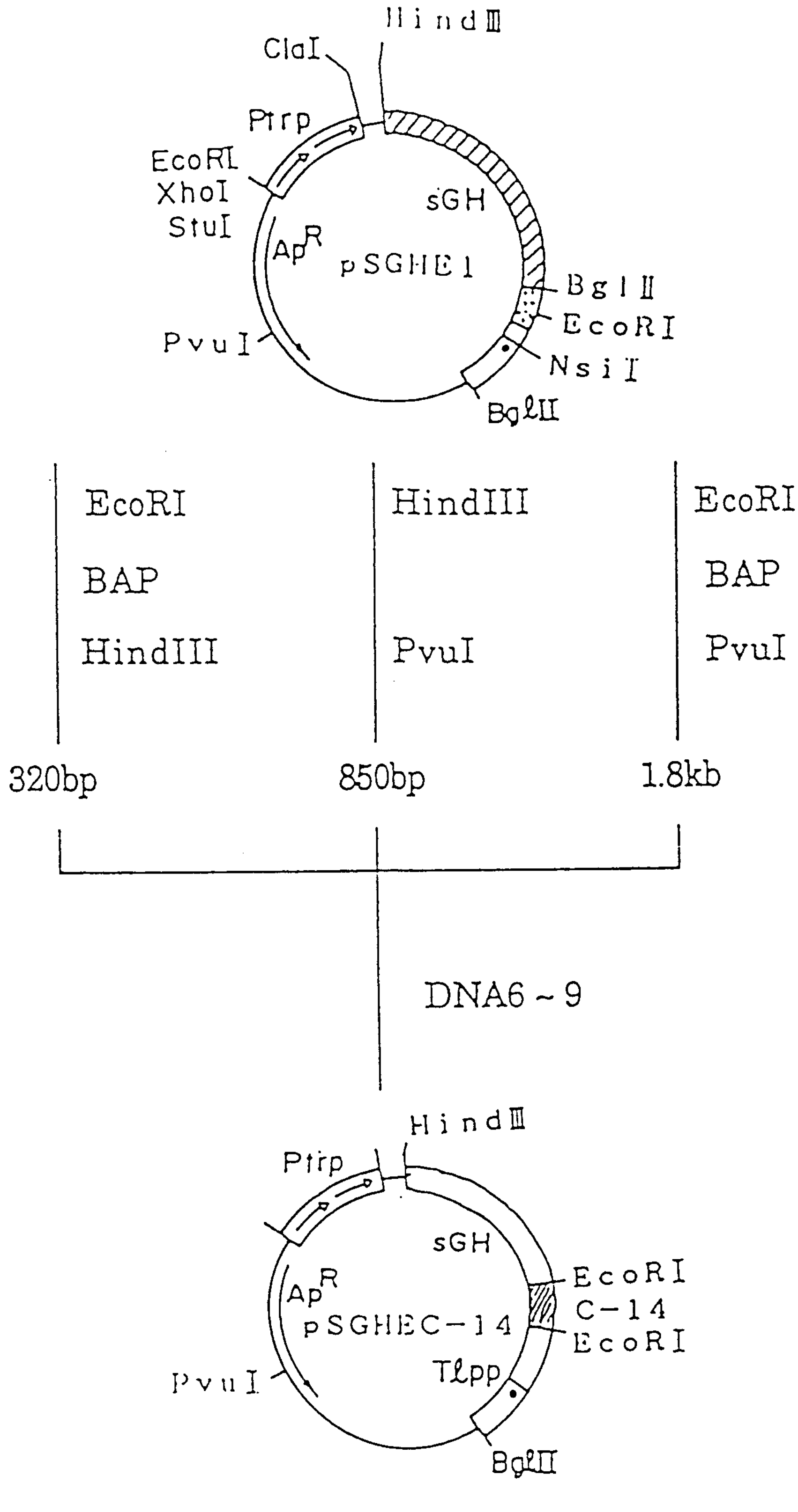
HindIII
ClaI
Ptrp
EcoRI
XhoI
StuI
sGH
ApR
pSGHE1
BglII
EcoRI
NsiI
PvuI
BglII
EcoRI
BAP
HindIII
HindIII
PvuI
EcoRI
BAP
PvuI
320bp
850bp
1.8kb
DNA6 ~ 9
HindIII
Ptrp
sGH
EcoRI
C-14
EcoRI
ApR
pSGHEC-14
PvuI
Tℓpp
BglII

Fig. 4

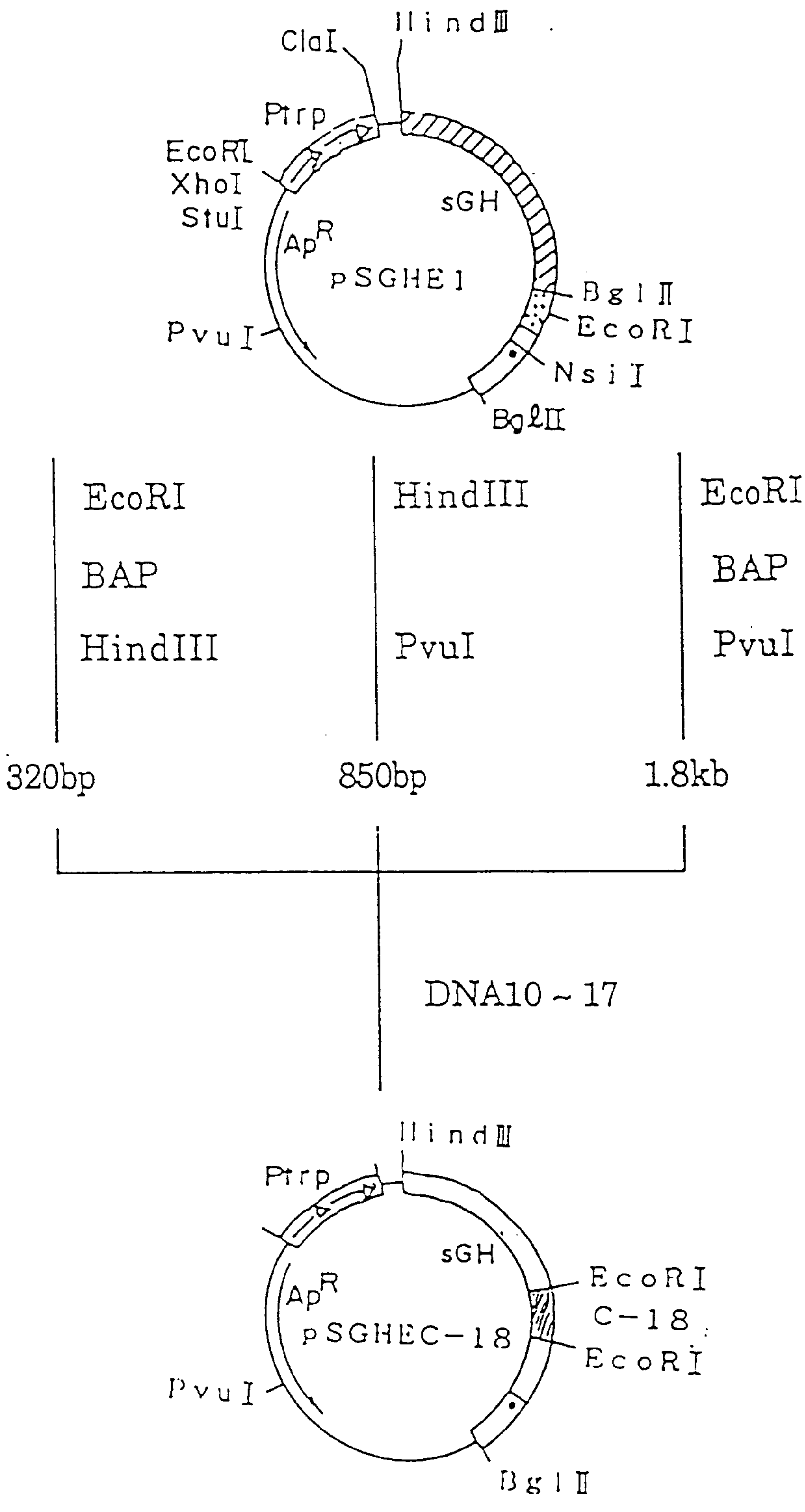
HindIII
ClaI
Ptrp
EcoRI
XhoI
StuI
sGH
ApR
pSGHE1
BglII
EcoRI
NsiI
PvuI
BglII
EcoRI
BAP
HindIII
HindIII
PvuI
EcoRI
BAP
PvuI
320bp
850bp
1.8kb
DNA10 ~ 17
HindIII
Ptrp
sGH
ApR
pSGHEC-18
EcoRI
C-18
EcoRI
PvuI
BglII

Fig. 5

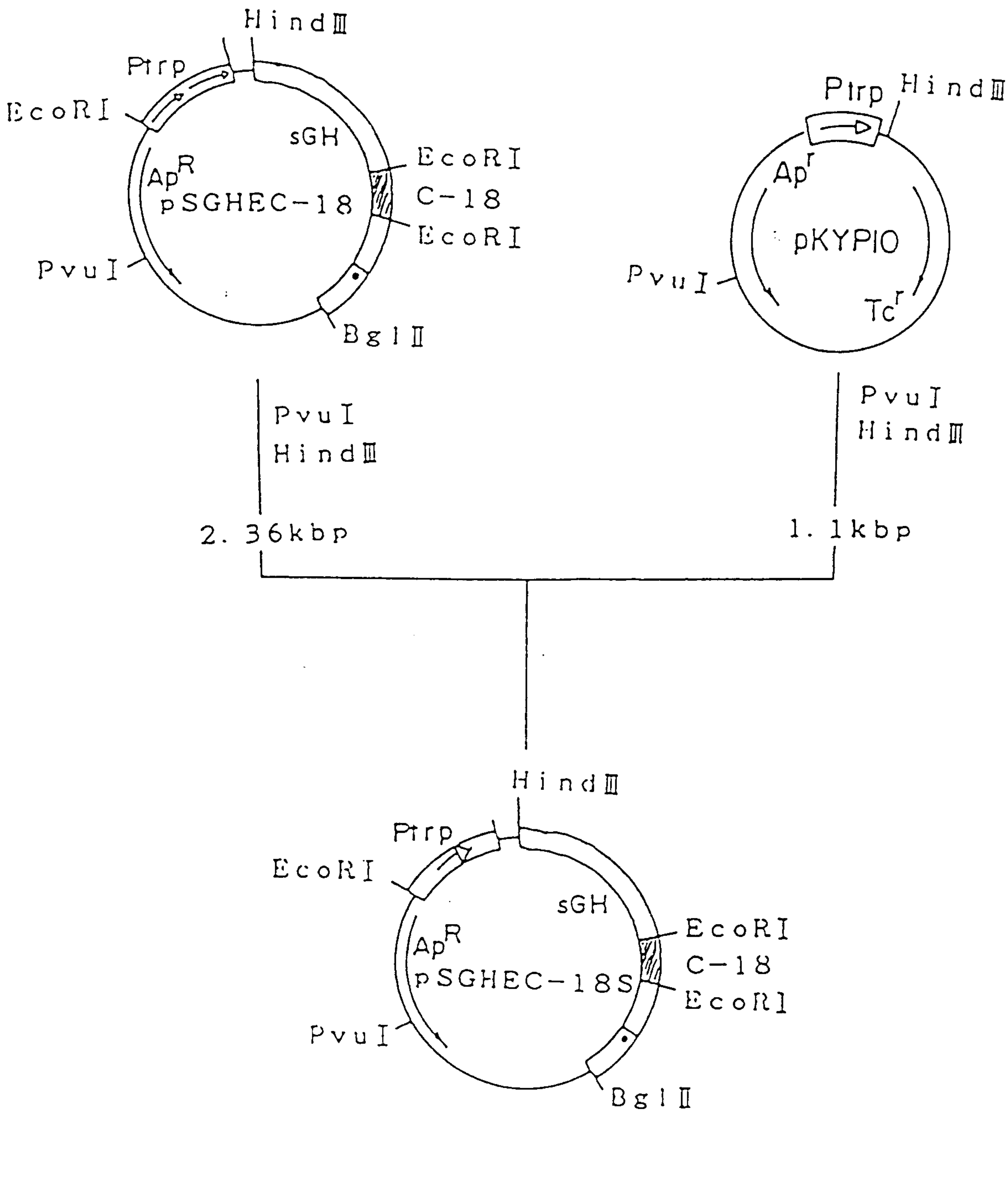
HindIII
Ptrp
EcoRI
sGH
EcoRI
ApR
pSGHEC-18
C-18
EcoRI
PvuI
BglII
Ptrp
HindIII
Apr
pKYP10
PvuI
Tcr
PvuI
HindIII
PvuI
HindIII
2.36kbp
1.1kbp
HindIII
Ptrp
EcoRI
sGH
EcoRI
ApR
C-18
pSGHEC-18S
EcoRI
PvuI
BglII

Fig. 6

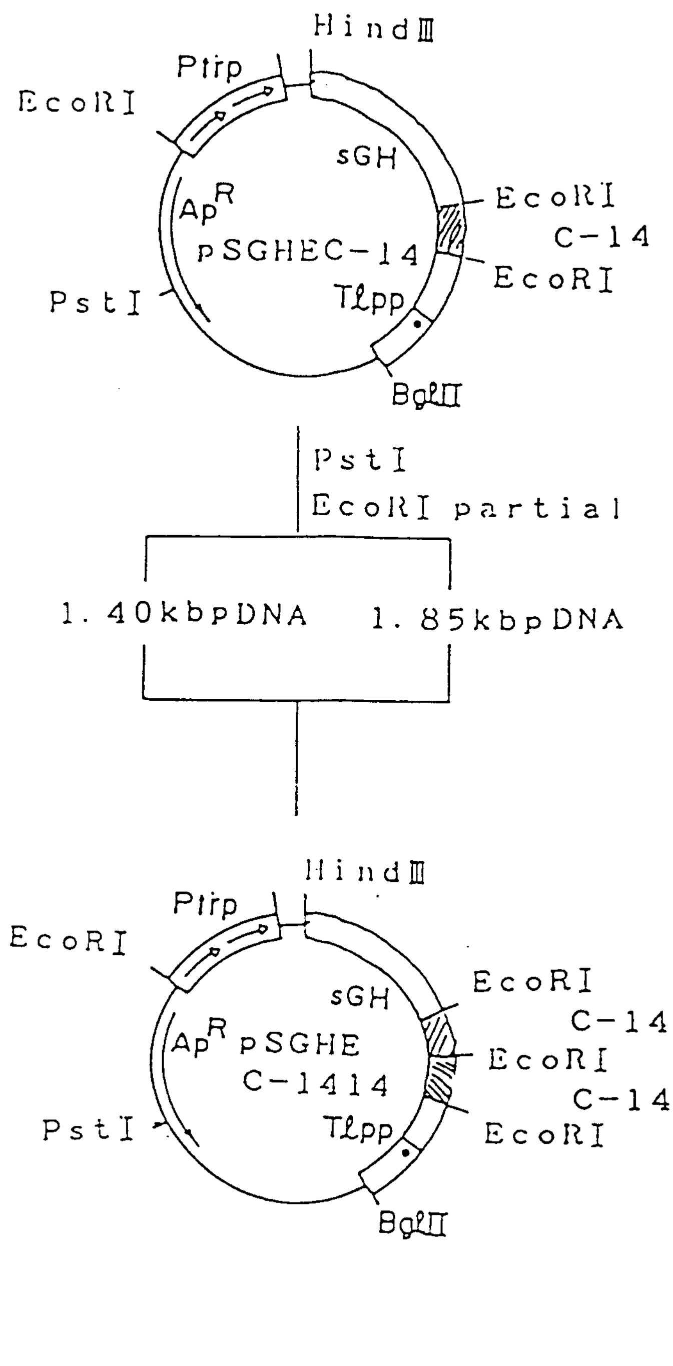
HindIII
Ptrp
EcoRI
sGH
ApR
pSGHEC-14
EcoRI
C-14
EcoRI
PstI
TLpp
BglII
PstI
EcoRI partial
1.40kbpDNA
1.85kbpDNA
HindIII
Ptrp
EcoRI
sGH
ApR pSGHE
C-1414
EcoRI
C-14
EcoRI
C-14
EcoRI
PstI
TLpp
BglII

Fig. 7

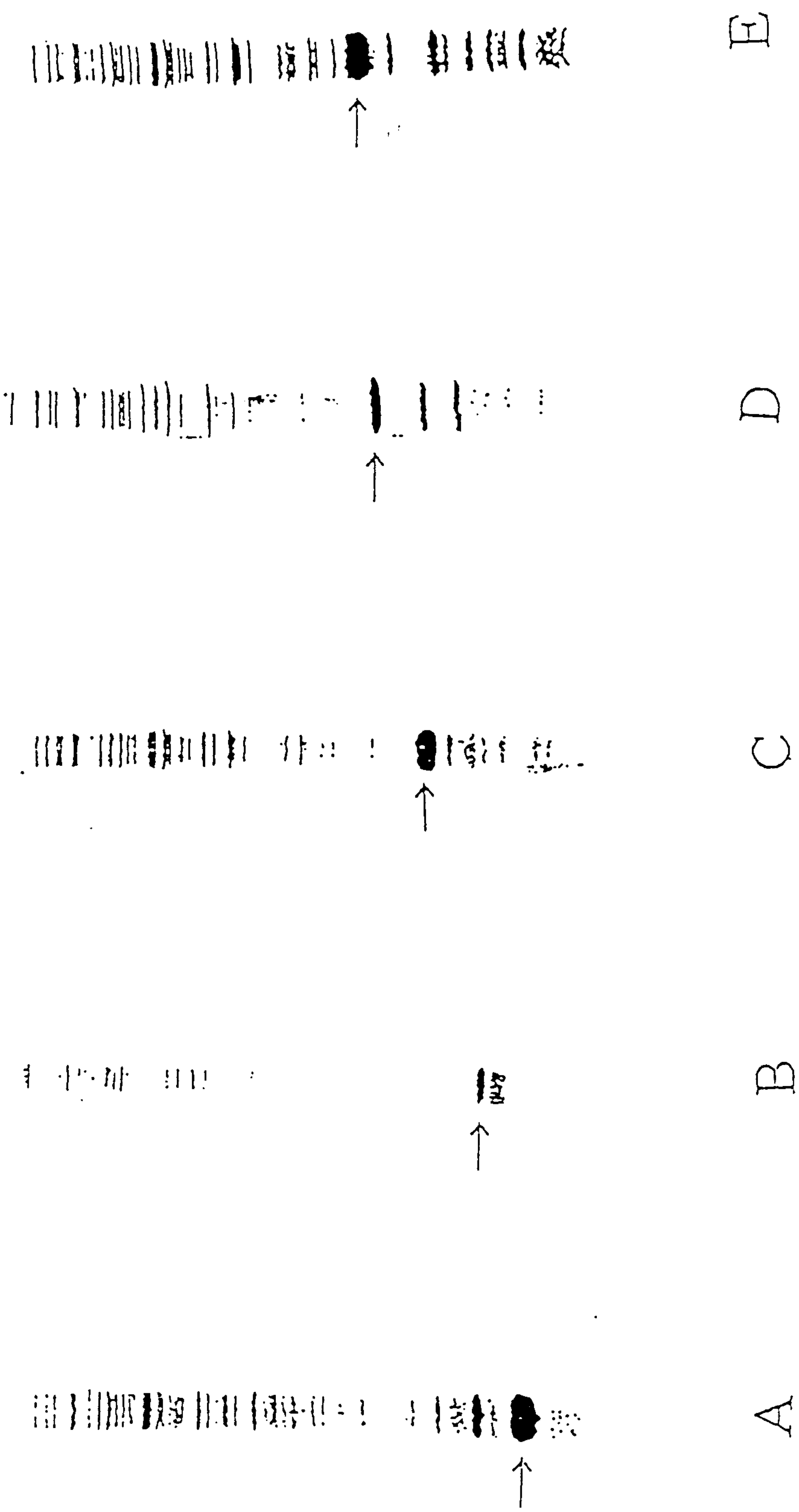

Fig. 8

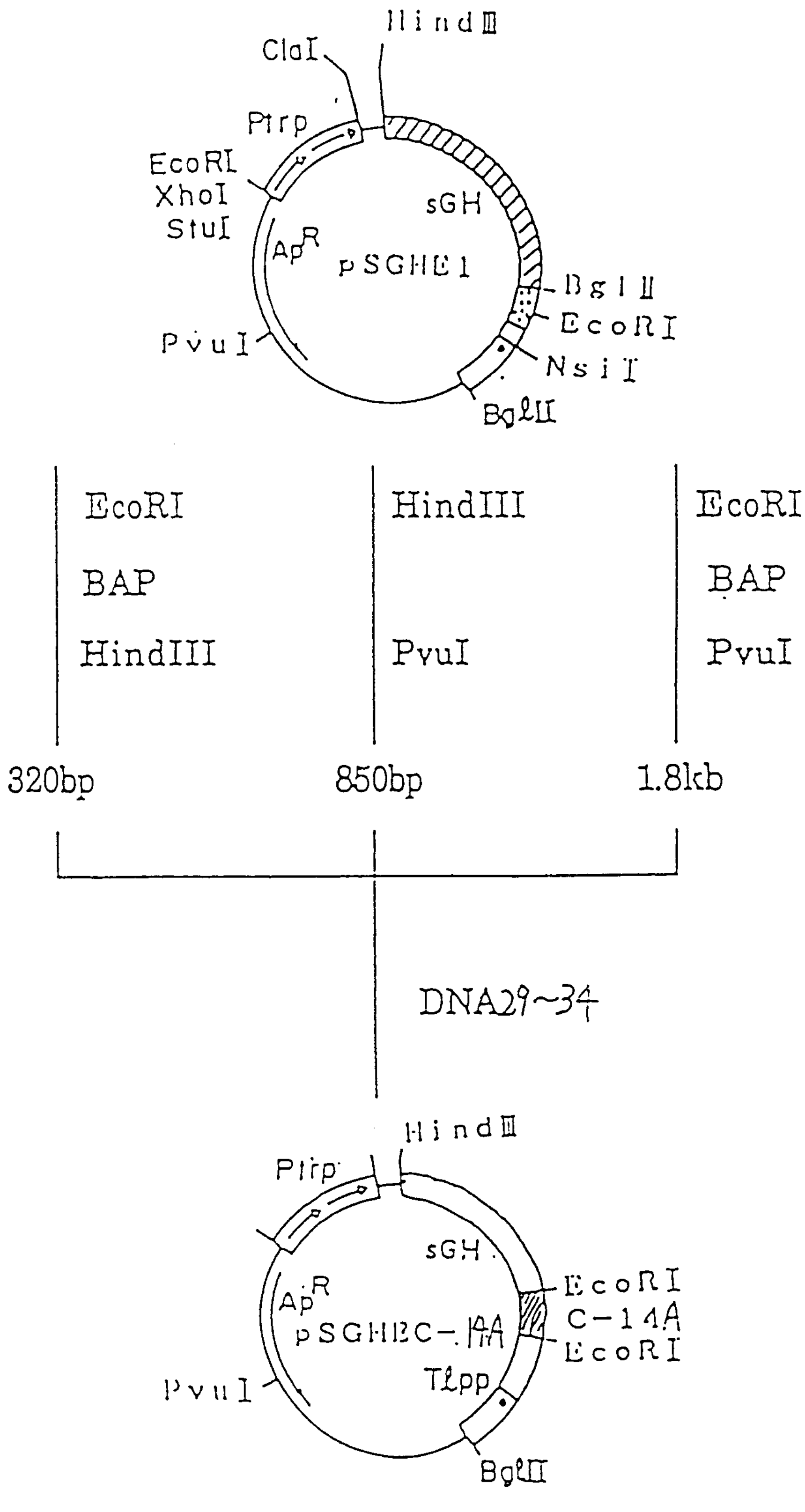
HindIII
ClaI
Ptrp
EcoRI
XhoI
StuI
sGH
ApR
pSGHE1
BglII
EcoRI
NsiI
PvuI
BglII
EcoRI
BAP
HindIII
HindIII
PvuI
EcoRI
BAP
PvuI
320bp
850bp
1.8kb
DNA29~34
HindIII
Ptrp
sGH
ApR
pSGHEC-14A
EcoRI
C-14A
EcoRI
TLpp
PvuI
BglII

Fig. 9

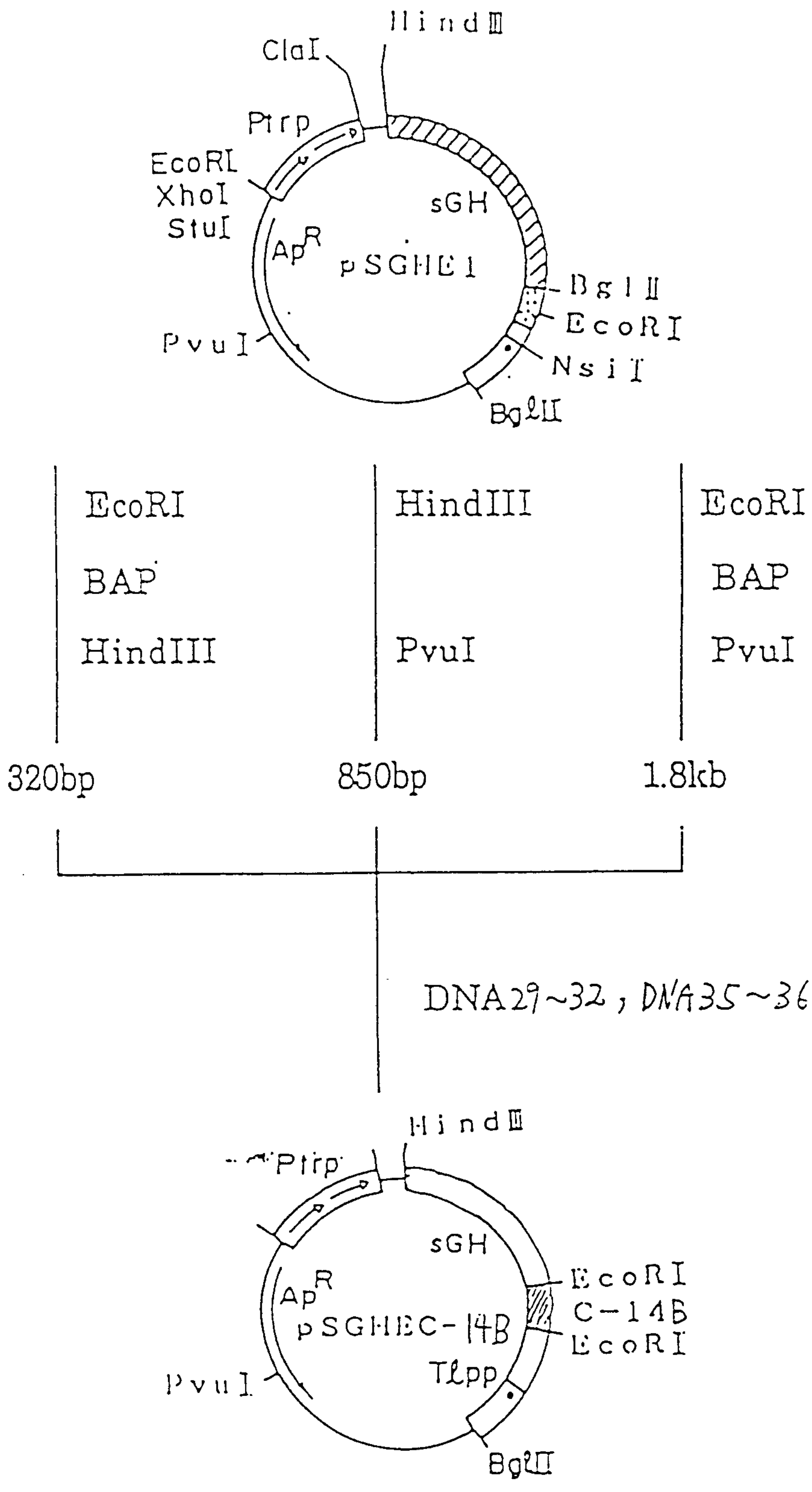
HindIII
ClaI
Ptrp
EcoRI
XhoI
StuI
sGH
Ap^R
pSGHE1
BglII
EcoRI
NsiI
PvuI
BglII
EcoRI
BAP
HindIII
HindIII
PvuI
EcoRI
BAP
PvuI
320bp
850bp
1.8kb
DNA29~32, DNA35~36
HindIII
Ptrp
sGH
Ap^R
pSGHEC-14B
EcoRI
C-14B
EcoRI
Tlpp
PvuI
BglII

Fig. 10

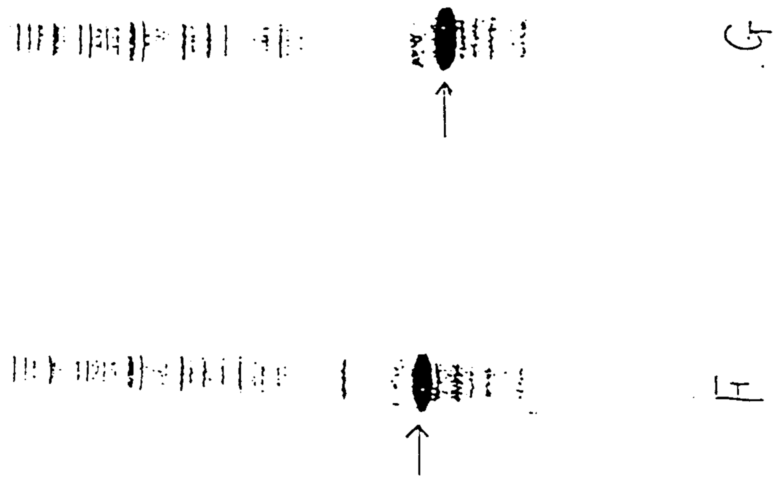

# INTERNATIONAL SEARCH REPORT

International Application No PCT/JP91/00376

**I. CLASSIFICATION OF SUBJECT MATTER** (if several classification symbols apply, indicate all) [6]

According to International Patent Classification (IPC) or to both National Classification and IPC

Int. $Cl^5$ C07K15/12, C12N15/62, C12P21/00, G01N33/576//
(C12P21/00, C12R1:19)

**II. FIELDS SEARCHED**

Minimum Documentation Searched [7]

| Classification System | Classification Symbols |
|---|---|
| IPC | C07K15/12, C12N15/51, 15/62, C12P21/00-21/02, G01N33/576 |

Documentation Searched other than Minimum Documentation to the Extent that such Documents are Included in the Fields Searched [8]

Biological Abstracts Data Base (BIOSIS)

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X/Y | WO, A1, 89/4669 (Chiron Corporation), June 1, 1989 (01. 06. 89), & JP, A, 2-500880 & EP, A1, 318216 | 1, 5, 10/ 2-4, 6-9, 11-15 |
| Y | Science, Vol.244, (1989), Qui Lim Choo et al. "Isolation of a cDNA clone derived from a blood-borne non-A, non-B viral hepatitis genome" p.359-362 | 1-15 |
| Y | JP, A, 61-15699 (Kyowa Hakko Kogyo Co., Ltd.), January 23, 1986 (23. 01. 86), & EP, A2, 166444 & JP, A, 61-93196 & JP, A, 61-93197 & JP, A, 61-210100 & US, A, 4689402 | 1-15 |
| Y | JP, A, 54-145289 (Genentech, Inc.), November 13, 1979 (13. 11. 79), & EP, A1, 1929 & DE, A, 2848051 | 1-15 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| June 13, 1991 (13. 06. 91) | July 1, 1991 (01. 07. 91) |
| International Searching Authority | Signature of Authorized Officer |
| Japanese Patent Office | |